# EUROPEAN PATENT APPLICATION

(11) **EP 2 213 367 A1**
(43) Date of publication of application: **04.08.2010**
(21) Application number: 08841333.1
(22) Date of filing: 20.10.2008
(51) Int. Cl.: B01J 8/02, C07C 41/09, C07C 43/04, C07C 1/04, C01B 3/16

(54) **A COMPOSITE REACTION APPARATUS AND THE CHEMICAL PRODUCTION METHOD USING THE SAME**

(30) Priority: 19.10.2007 CN 200710181403; 13.12.2007 CN 200710198891; 29.02.2008 CN 200810082162
(71) Applicant: Lou, Ren, Zhejiang 310012 (CN)
(72) Inventor: LOU, Shoulin, Hangzhou Zhejiang 310012 (CN)
(74) Representative: Altenburg, Udo
(86) International application number: PCT/CN2008/072750
(87) International publication number: WO 2009/052764

(57) **Abstract**

A composite reaction apparatus, comprising: a capped cylindrical shell (1), a reaction gas inlet (2) and a reaction gas outlet (3) on the shell (1), a catalyst layer (4), a porous gas distributor (7), a porous gas collection plate (8) and heat-exchange tubes (5) in the catalyst layer (4) for removing heat with cooling medium, and vapor tanks (61, 62) connected to pipes (41, 42) with vaporization pressure adjusting values, wherein there are more than two catalytic reaction zones (411, 412......) along the flowing direction of the reaction gas in the catalyst layer (4), wherein at least two of the reaction zones comprise groups of heat-exchange tubes (501, 502) to form heat-exchange reaction zones, wherein the groups of heat-exchange tubes (501, 502) are connected via inlet pipes (302, 304) and outlet pipes (301, 303) for cooling medium to the vapor tanks (61, 62) capable of independently adjusting vaporization pressure, forming circulating loops of the cooling medium having different vaporization pressures and temperatures, wherein the circulating loops can comprise recycle pumps to enhance circulation as desired, so that the reaction gas reacts at different heat exchanging rates sequentially in the catalytic reaction zones to obtain desirable constant temperature or optimal temperature distribution.

## Description

### Technical Field

The present invention, which belongs to the technical field of chemical engineering, relates to an apparatus for catalytic reaction, which apparatus can be used in fluid catalytic reactions and heat exchange processes, and is particularly suitable for strong e3xothermic reactions such as synthesizing methanol or dimethylether, methanation reaction, Fischer-Tropsch reaction, oxidizing H₂S, CO transformation, synthesizing ammonia and the like. The present invention further relates to methods for synthesizing dimethylether or hydrocarbons, relates to method for producing hydrocarbons by Fischer-Tropsch reaction from syngas, relates to method for producing natural gas by the methanation of syngas using the apparatus, and relates to methods of chemical-power co-production using the apparatus.

### Background Art

In order to enhance the reaction efficiency of certain strong exothermic reactions such as those in synthesis of methanol, preparation of dimethylether from syngas, methanation reaction, Fischer-Tropsch reaction and oxidizing H₂S, it is necessary to remove reaction heat during the reaction. For example, during the process for removing reaction heat from the reaction tube in a Lurgi tube-shell tower for synthesizing methanol by cycling water in the shell, if Q_{R} represents the reaction heat and Q_{E} represents the heat transferred by a cooling agent, isothermal reaction will be achieved when Q_{R}= Q_{E}. The heat transferred may be expressed by the equation, Q_{E} =KFΔT, wherein K is a heat transfer coefficient, F is a heat transfer area, and ΔT is a temperature difference between the reaction gas and the cooling agent. Current theories on designing catalytic reactors (Catalytic Reaction Engineering Bingchen Zhu Ed. , China Petrochemical Press, Feb. 2000, P218-P219) point out: "Since a small temperature difference between a heat carrier and a catalytic bed is desirable on the one hand, and a large quantity of heat emitted by the reaction has to be removed on the other hand, a large heat transfer area and a large heat transfer coefficient are required", and "the temperature change in the cooling agent may be omitted when the cooling agent, such as pressurized water, outside the tube is vaporized or a salt is molten", which means that, in the design of a tube-shell reactor, the temperature of the cooling agent is considered unchanged during the heat-exchange of the whole catalytic bed. However, increase of heat transfer area F is restricted structurally. For a tube-shell reactor, the specific cooling area or the heat-exchange area of one cubic meter of catalyst has been up to over 120 square meters, which is hard to be further increased. It is usual to increase the flow rate and linear velocity of the input gas to timely carry the reaction heat out of the tower to prevent "over temperature " or "temperature runaway". Therefore, it is necessary to utilize a circulating gas, which is 5-10 times (i.e., circulating ratio) more than the raw gas, to reduce the amount of the raw gas in the total gases entering the synthesis tower, otherwise, the intensive heat generated by the rapid reaction would deactivate the catalyst.

However, a higher circulating ratio will entail increased investment in equipment and piping for methanol synthesis, and increase driving force and energy consumption. It has been manifested upon calculation that, in the process of producing syngas from coal by the gasification of Shell powdered coal or Texaco coal water slurry, the reaction gas exiting the synthesis tower will comprise up to 50% of methanol or more if a low circulating ratio is used, while the reaction gas exiting a conventional methanol synthesis tower will comprise only about 5% methanol, just one tenth of the former. As indicated in literature, "the content of methanol at the outlet of the tower is 5%-6% when the circulating ratio is 5 in the Lurgi process; and that content is 3%-4% when the circulating ratio is 10 in the ICI process" (Methanol Entering, Song Weiduan et al. ed., Chemical Industry Press, 1991. 1, 8178). High circulating ratio increases the difficulty in scaling up corresponding industrial apparatus as well as investment.

In addition, due to the large quantity of reaction heat which is difficult to be timely removed, huge volume of circulating gas is needed in one-step preparation of dimethylether, preparation of gasoline from syngas via methanol, synthesis of natural gas from syngas by Fischer-Tropsch reaction and chemical-power co-production, which are economically unfavorable in such a case and thus difficult to be industrialized.

Thus, there is a need to provide an energy-efficient apparatus for reactions having low circulating ratio and high reaction rate with concentrated raw gas.

There is also a need for methods for synthesis of dimethylether, synthesis of hydrocarbons, preparation of hydrocarbons by Fischer-Tropsch reaction, preparation of natural gas by methanation of syngas and chemical-power co-production using said apparatus.

### Summary of the Invention

One object of the present invention is to provide an energy-efficient apparatus for reactions having low circulating ratio and high reaction rate with concentrated raw gas, as well as cost-efficient methods for synthesis of methanol, dimethyl ether, hydrocarbons and chemical-power co-production.

One aspect of the present invention is to provided a composite reaction apparatus, comprising:
a reaction device 9 comprising a capped cylindrical shell 1, a reaction gas inlet 2 and a reaction gas outlet 3 on the shell 1, a catalyst layer 4, a porous gas distributor 7, a porous gas collection plate 8 and heat-exchange tubes 5 in the catalyst layer 4 for removing heat by cooling agent therein; and
vapor tanks 61, 62 connected to pipes 41, 42 having vaporization pressure adjusting values;
wherein there are more than two catalytic reaction zones 411, 412...... along the flowing direction of the reaction gas in the catalyst layer 4, at least two of the reaction zones comprise groups of heat-exchange tube 501, 502 to form heat-exchange reaction zones,
wherein one group of heat-exchange tube 501 is connected to the vapor tank 61 via an inlet pipe 302 and an outlet pipe 301 for cooling agent, said vapor tank 61 is capable of independently adjusting its vaporization pressure, or one group of heat-exchange tube 502 is connected to the vapor tank 62 via an inlet pipe 304 and an outlet pipe 303 for cooling agent, said vapor tank 62 is capable of independently adjusting its vaporization pressure, forming circulating loops of the cooling agent having adjustable vaporization pressure and temperature; and
wherein the circulating loops can optionally comprise recycle pumps to enhance circulation as desired;
so that the reaction gas proceeds reaction at different heat exchanging rates sequentially in the catalytic reaction zones to obtain desirable constant temperature or optimal temperature distribution.

In a preferred embodiment of the present invention, each group of heat-exchange tube 501, 502, 503 in the apparatus can be connected to the vapor tank 61 1 or the vapor tank 62 respectively as desired. For example, the vapor tank 62 is connected to the inlet pipes 302, 304, 306 of the heat-exchange tube groups 501, 502, 503 in the reaction device 9 via a water pipe 72, a water pump 82 and water pipes 102, 104, 106 having valves respectively, while the outlet pipes 301, 303, 305 of the heat-exchange tube groups 501, 502, 503 are fluidly communicated with vapor tank 62 via vapor-water pipes 101, 103, 105 having valves, respectively, and a vapor-water pipe 92. Every pair of the valves in the water pipes for fluidly communicating with the vapor tank 62 (e.g. vapor-water pipe 101 and water pipe 102, vapor-water pipe 103 and water pipe 104, as well as vapor-water pipe 105 and water pipe 106) can be used to control the flow rate of the water pipe flowing into the capped cylindrical shell 1 or stop same. The vapor tank 61 is connected to the inlet pipes 302, 304, 306 of the heat-exchange tube groups 501, 502, 503 in the reaction device 9 via a water pipe 71, a water pump 81 and water pipes 202, 204, 206 having valves, respectively, while the outlet pipes 301, 303, 305 of the heat-exchange tube groups 501, 502, 503 are fluidly communicated with a vapor-water pipe 91 and connected to the vapor tank 61 via vapor-water pipes 201, 203, 205 having valves, respectively. Every pair of valves in the water pipes for fluidly communicating with the vapor tank 61 (e.g. vapor-water pipe 201 and water pipe 202, vapor-water pipe 203 and water pipe 204, vapor-water pipe 205 and water pipe 206) can be used to control the flow rate of the water pipe flowing into the capped cylindrical shell 1 or stop same. The valves are adjusted such that each group of heat-exchange tube is fluidly communicated with one vapor tank.

The composite reaction apparatus as described above can be used mainly in such strong exothermic reactions as in the preparation of methanol from syngas comprising H₂, CO and CO₂, synthesis of dimethyl ether, preparation of liquid hydrocarbons by Fischer-Tropsch reaction, methanation reaction, preparation of hydrocarbons (such as ethylene, propylene, gasoline, etc.) from syngas via methanol, oxidation of o-xylene to prepare phthalic anhydride, oxidation of ethylene to synthesize ethylene oxide, hydrogenation of acetylene to prepare ethylene, hydrogenation of benzene to prepare cyclohexane, CO transformation, synthesis of ammonia, oxidation of hydrogen sulfide to prepare sulfur, etc..

In another aspect of the invention, there is provided a method for preparing dimethylether from a syngas, comprising:
catalytically synthesizing methanol from the syngas in the apparatus described above;
dehydrating methanol in a methanol dehydration reactor 4002 to form dimethyl ether-containing reaction gas;
separating dimethyl ether, methanol and water from the reaction gas upon heat-exchange; and
recycling and combining the remaining reaction gas, except a small amount thereof being relieved, with the starting syngas for further synthesis of dimethyl ether.

The molecular ratio of the recycled gas to the starting syngas is less than 5, preferably less than 4, the pressure in the synthesis of methanol and dimethyl ether is 5 MPa or higher, and the volume of methanol in the reaction gas after the step of catalytically synthesizing methanol is more than 7% by volume, preferably more than 10%.

In another aspect of the present invention, there is provided a method for preparing hydrocarbons from a syngas, comprising:
pressurizing the syngas comprising hydrogen, carbon monoxide and the like;
synthesizing methanol or synthesizing a mixture of methanol and dimethyl ether in the methanol synthesis reactor 4001 consisting of the apparatus of the present invention;
dehydrating the obtained methanol or the mixture of methanol and dimethylether, without proceeding a step of separation, in a dehydration reactor 4002 containing a dehydration catalyst such as γ -Al₂O₃ (alumina) or molecular seive ZSM-5 at the same level of pressure as that in the synthesis step above, obtaining hydrocarbons;
separating the reaction gas by cooling, obtaining hydrocarbons product, water and unreacted gas; and
sending the unreacted gas to a gas/steam Integrated Gasification Combined Cycle (IGCC), or recycling the unreacted gas, except a small amount thereof being relieved, as a part of the starting material, the molecular ratio of the recycled unreacted gas to the starting syngas is less than 4, preferably less than 2.

In another aspect of the present invention, there is provided a method for preparing hydrocarbons from a syngas by Fischer-Tropsch reaction, comprising:
pressurizing the syngas comprising hydrogen, carbon monoxide and the like;
proceeding a Fischer-Tropsch reaction in a Fischer-Tropsch reactor 4001 consisting of the apparatus of the present invention, obtaining relatively heavy hydrocarbons having various fractions and water, etc.;
separating the reaction gas flowed out from the Fischer-Tropsch reactor 4001 by cooling, or flowing the reaction gas from the Fischer-Tropsch reactor 4001 to a hydrocarbon modification reactor 4002 filled with molecular sieve to modify the hydrocarbons and then separating the obtained reaction gas by cooling to obtain hydrocarbons, water and unreacted gas; and
flowing the unreacted gas to a gas/steam Integrated Gasification Combined Cycle (IGCC), or recycling the unreacted syngas, except for a small amount thereof being relieved, to proceed a further Fischer-Tropsch reaction together with a fresh syngas.

In another aspect of the present invention, there is provided a method for preparing synthetic natural gas by the methanation of coal-derived syngas or coke oven gas, comprising a step of proceeding a methanation reaction of H₂, CO, and CO₂ in the starting syngas to produce methane at the presence of a methanation catalyst in an external cooling type heat-exchange reactor consisting of the apparatus of the present invention, the reaction heat of the methanation reaction can be used to form a high-pressure steam.

In another aspect of the present invention, there is provided a method of chemical-power co-production, comprising the steps of:
gasifying a carbon-containing starting material with a gasification agent such as water, oxygen, carbon dioxide and the like, obtaining a syngas;
flowing the syngas the apparatus of the present invention to produce chemical products such as methanol, dimethyl ether or other hydrocarbons;
flowing unreacted gas as a fuel to a gas/steam integrated recycle power generator system; and
sending the steam produced during gasifying and purifying syngas and in chemical synthesis to the steam power generator.

In another aspect of the present invention, there is provided a method for synthesizing ammonia from a hydrogen- and nitrogen-containing synthetic gas, comprising a step of synthesizing ammonia at a pressure of 6-20 MPa and a temperature of 310-450°C in the apparatus of the present invention at the presence of an iron-family or ruthenium-family ammonia synthesis catalyst, such a process produces a steam of 8-15 MPa as a byproduct; wherein the synthesis step is carried out at a constant or optimal temperature by using two or more reaction zones in the apparatus, the vaporization pressure and temperature in the heat-exchange tubes in each of the reaction zone can be adjusted independently. The reaction equation of ammonia synthesis is as follows:

1/2 N₂ + 1.5 H₂ = NH₃ + 46.2KJ/mol.

The conventional technology of ammonia synthesis tends to produce a co-produced high-pressure steam. For example, an energy-saving process disclosed by Brown Co. utilizes two ammonia synthesis towers, and the reaction gas exiting the towers co-produces a high-pressure steam; EP 0994072 to KBR Co., USA has disclosed a process for synthesizing ammonia by utilizing a tube-shell reactor containing a ruthenium family catalyst, however, which reactor is only suitable for synthesizing ammonia from the relief gas of the present apparatus due to restriction on heat removal capability as described above. Casale Co., Swiss has developed a plate type water-cooling ammonia synthesis tower operating at a constant temperature (see Nitrogen Syngas No. 291) since 2005. However, all of the apparatuses disclosed above are axial towers, suffering from both restriction of heat-exchange capability and large pressure drop in the towers.

In another aspect of the present invention, there is provided a method for transforming carbon monoxide in syngas, comprising to transform carbon monoxide at a pressure of 1-18 MPa and a temperature of 280-430°C at the presence of an iron-chromium family middle- or high- temperature shift catalyst or a sulfur resistant catalyst in the apparatus of the present invention, alternately, to transform carbon monoxide at a pressure of 1-18 MPa and at a temperature of 180-300°C at the presence of a copper family low-temperature shift catalyst in the apparatus of the present invention; the reaction co-produces a steam of 1-15 MPa. The reaction equation of carbon monoxide transformation is as follows:

CO + H₂O = CO₂ + H₂ + 41.19KJ/mol.

In the conventional process for the transformation of carbon monoxide, heat-exchange is carried out between two reaction stages of a multistage adiabatic reaction used for carbon monoxide transformation. An isothermal transformation oven designed by Linde Co ( See The Chemistry of Ammonia Synthesis and Carbon, Li Qiongjiu ed., Sichuan Science and Technology Press, Aug. 2000) comprises a reactor equipped with a water-cooling helix tube, the temperature of which was adjusted by controlling the pressure of the co-produced steam. However, such an oven is restricted by its heat-exchange capability, resulting in the temperature not being shifted according to the requirement of the catalytic reaction or the temperature cannot be adjusted independently.

Various aspects of the present invention will be described in detail with reference to the accompanied drawings.

### Brief Description of the Drawings

Fig. 1 is a schematic drawing of an apparatus comprising two vapor tanks and a horizontal water-cooling reaction device having U-shaped heat-exchange tubes;
Fig. 2 is a schematic drawing of an apparatus comprising two vapor tanks and a horizontal water-cooling reaction device;
Fig. 3 is a schematic drawing of an apparatus comprising two vapor tanks and a horizontal water-cooling reaction device having adjusting valves therebetween;
Fig. 4 is a schematic drawing of an apparatus comprising two vapor tanks and a vertical water-cooling reaction device having adjusting valves therebetween;
Fig. 5 is a schematic drawing of a vertical reaction device having multi-groups of heat-exchange tubes;
Fig. 6 is a schematic drawing of a horizontal reaction device having multi-groups of heat-exchange tubes therein;
Fig. 7 is a schematic flow chart for the synthesis of dimethyl ether from syngas by one-step;
Fig. 8 is a schematic flow chart for the synthesis of hydrocarbons from syngas via methanol by one-step or the synthesis of hydrocarbons from syngas by Fischer-Tropsch reaction;
Fig. 9 is a schematic drawing of an apparatus comprising three vapor tanks and a reaction device having three groups of heat-exchange tubes;
Fig. 10 is a schematic flow chart of the combination of synthesizing hydrocarbons by Fischer-Tropsch reaction and IGCC;
Fig. 11 is a schematic flow chart for the preparation of natural gas by methanation reaction of syngas according to one embodiment of the present invention;
Fig. 12 is a schematic drawing of a co-production system for producing chemical products and electricity power by using coal as the starting material; and
Fig. 13 is a schematic flow chart of the production of chemical products from syngas by using multi-groups of water-cooling reaction devices.

### Detailed Description of the Invention

The technical schemes of the invention will be described in detail with reference to the accompanied drawings.

As used herein, the term "reaction gas" or "reacted gas" refers to those that flowing in the inlet of the reaction device, proceeding a reaction in the reaction device and/or flowing out of the outlet of the reaction device.

Conventionally, catalytic reactions are not proceeded at the same rate at different parts of the catalyst layer. Generally, since the reaction at the front part of a reaction device is relatively far from equilibrium, its reaction rate will be faster and more reaction heat will be released, while reaction at the end part of the reaction device approaches equilibrium, its reaction rate will be decelerated and less reaction heat will be released. However, in the conventional tube-shell water-cooling reaction device, the cooling agent utilized has an even temperature in all heat-exchange tubes. If the temperature of the cooling agent is decreased to enlarge the temperature difference ΔT between the cooling agent and the reaction gas so as to meet the requirement of removing the intensive reaction heat Q_{R} produced under the high reaction rate in the front part of the reaction device, since the reaction heat Q_{R} at the end part of the reaction device is lower than that in the front part of the reaction device, Q_{E} (Q_{E} = KFΔT, see the above) will be more than Q_{R} at the end part of the reaction device, resulting in the reaction temperature in the end part being over-decreased, as a result, the reaction rate will be gradually decelerated and the reaction will finally be stopped when the catalyst loses its activity due to too low a temperature. Thus, it is difficult to allow the reaction to be proceeded at optimal temperature in all parts of the reaction device.

In order to resolve this technical problem in the prior art, the present invention provides a technical solution which utilizes a cooling agent having various temperatures for different reaction zones in a reaction device, so that the heat removed at one part can be equal to the reaction heat produced at that part. Specifically, the reaction device can be divided into several reaction zones along the flowing direction of the reaction gas in the catalyst layer, and each of the reaction zone can be heat exchanged with a cooling agent flowing in a group of heat-exchange tube, independently.

For example, with respect to the reaction for synthesizing methanol, since the reaction temperature is from 180°C to 300°C and since the heat for vaporizing a liquid cooling agent is much higher than that for warming same, a pressurized hot water may be used as the cooling agent at that temperature. With respect to even higher reaction temperature, a low volatility mineral oil, heat conductive oil or molten salts may be used as the cooling agent to carry the reaction heat to a vapor tank through a circulating loop, the reaction heat will be recovered in the vapor tank by producing a steam. When water is used as the cooling agent, said water will remove the reaction heat during synthesis of methanol and become steam directly. If the catalyst for the synthesis of methanol is disposed within the heat-exchange tubes (such as in the case of Lurgi tube-shell reactor), such heat-exchange tubes will act as reaction tubes while water will be filled outside the reaction tubes. Alternatively, if the catalyst is disposed outside the heat-exchange tubes, the heat-exchange tubes will be water pipes (such as a water pipe type reaction device). The water pipe may be vertical, helical or horizontal (such as a horizontal water-cooling methanol tower).

The advantages of the present invention are:
a) first of all, different reaction zones comprise different group of heat-exchange tubes. Each group of heat-exchange tubes may be fluidly communicated with a vapor tank having different vaporization pressures and temperatures depending on the requirements. For example, the cooling agent flowing in the heat exchange tubes at the front part of the reaction device has lower pressure and temperature to enlarge the temperature difference ΔT between the cooling agent and the reaction gas to enhance the heat exchange, resolving the technical problem of the reaction in the front part being too fast and producing more reaction heat. In addition, since the catalytic reaction rate will be decelerated and the reaction heat will be reduced at the end part of the reaction device, increasing the pressure and temperature of the cooling agent flowing in the heat-exchange tubes at that part correspondingly can reduce the temperature difference ΔT, so as to prevent the reaction heat from being removed excessively, resulting in too low a reaction temperature.
b) secondly, the pressure in the vapor tank is adjustable. Along with the proceeding of the reaction, the activity of the catalyst at the front part of the reaction device will be decreased faster than that in the end part, resulting in the catalytic reaction zone for proceeding most of the reaction moving gradually towards the end part of the reaction device and the reaction heat at said reaction zone increasing gradually. At that time, the vaporization pressure of the cooling agent flowing in the heat-exchange tubes at said reaction zone can be reduced, or said heat-exchange tubes may be connected to the vapor tank having lower pressure, so that the cooling agent flowing in said heat exchange tubes at said reaction zone can be vaporized at low temperature and pressure to increase the temperature difference between the cooling agent and the reaction gas in said reaction zone and, in turn, to increase the heat-exchange rate.

The pressure in said heat-exchange tubes and vapor tanks may be from 0.2MPa to 16MPa, depending on the requirement. In the reaction zones 411, 412, the pressure difference between the heat-exchange tubes 501, 502 and the vapor tanks connected thereto is less than 15MPa.

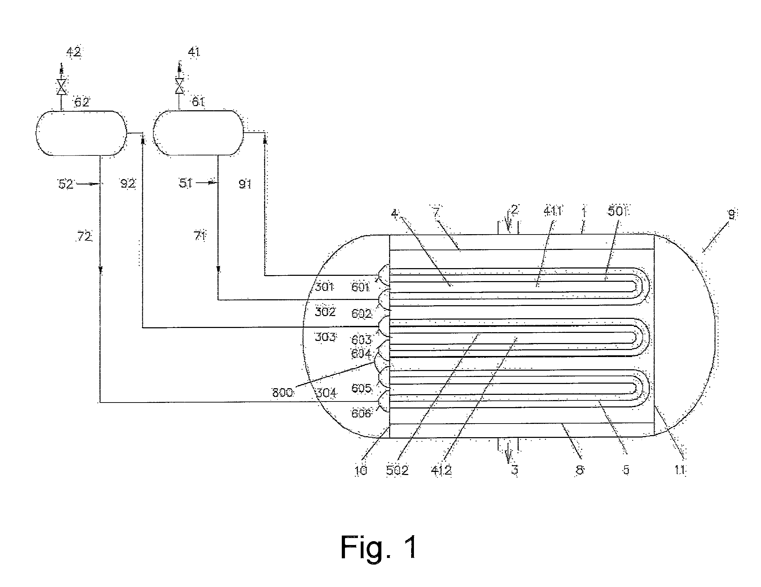

Fig. 1 shows an apparatus comprising two vapor tanks and a horizontal water-cooling reaction device having U-shaped heat-exchange tubes. The apparatus shown in the figure comprises a reaction device 9 and vapor tanks 61, 62 connected to pipes 41, 42 having vaporization pressure adjusting values; said reaction device 9 comprises a capped cylindrical shell 1, a reaction gas inlet 2 and a reaction gas outlet 3 on the shell 1, a catalyst layer 4, a porous gas distributor 7, a porous gas collection plate 8, U-shaped heat-exchange tubes 5 in the catalyst layer 4 for removing heat by utilizing a cooling agent, and partition plates 10, 11 at two ends of the heat-exchange tubes 5, respectively. There are two catalytic reaction zones, upper reaction zone 411 and lower reaction zone 412, along the flowing direction of the reaction gas in the catalyst layer 4, wherein two groups of heat-exchange tube 501, 502 form heat-exchange reaction zones.

Each group of the heat-exchange tube is composed of a plurality of U-shaped tubes, each of the U-shaped tube has two open ends, which are fluidly communicated with headers 601, 602, respectively. The inlet pipe 302 of the header 602 for the heat-exchange tube group 501 is connected to the vapor tank 61 via water pipe 71, and the outlet pipe 301 of the header 601 for the heat-exchange tube group 501 is connected to the vapor tank 61 via vapor-water pipe 91, forming a first circulating loop. The headers 604 and 605 for the heat-exchange tube group 502 are connected in series via a communicating pipe 800 to form a heat-exchange reaction zone 412. The inlet pipe 304 of the header 606 for the heat-exchange tube group 502 is connected to the vapor tank 62 via water pipe 72, and the outlet pipe 303 of the header 603 is connected to the vapor tank 62 via vapor-water pipe 92, so as to form a second circulating loop for a cooling agent having different vaporization temperature.

Water pumps 81, 82 may optionally be disposed in the water pipes 71, 72 of said circulating loops to enhance circulation. After entering the reaction device from the inlet 2, the reaction gas passes through the porous gas distributor 7, enters the volume between the partition plates 10 and 11 in the shell 1, and proceeds the reaction in various catalytic reaction zones at different heat exchanging rates. When water is used as the cooling agent, a steam will be co-produced directly, then the vapor tanks 61, 62 are pressure adjusting vapor tanks. When high reaction temperature and high cooling agent temperature, such as 300°C or more, are required, the cooling agent can be mineral oil, heat conducting oil or molten salts, then the vapor tanks 61, 62 are heat-exchange evaporators: the cooling agent carries the reaction heat into the vapor tanks 61, 62 and heat exchanges with water outside heat exchange tubes of the vapor tank, producing steam.

The inlet 2 and outlet 3 of the reaction gas in the reaction device may alternatively be disposed on the caps of the cylindrical shell 1, respectively. In addition, as shown in the figure, the water pipes 71, 72 can optionally be connected with pipes 51, 52, respectively, for supplementing water. Furthermore, both of heat-exchange tubes 5 and the porous gas collection plate 8 at the bottom may be supported correspondingly by supporting plates (which are not shown in the figure).

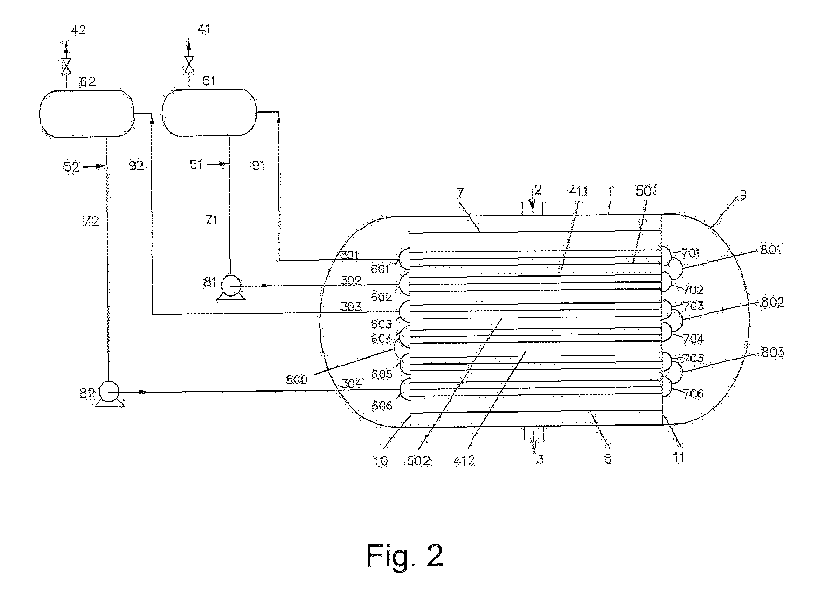

Fig. 2 shows an apparatus of one embodiment of the present invention. Those structures of the apparatus in Fig. 2 that are similar to those in Fig. 1 will not be repeated. The components different from those shown in figure 1 are:
(a) there are water pumps 81, 82 located in the pipes for connecting inlet pipes 302, 304 of the groups of heat-exchange tube 501, 502 to the vapor tanks 61, 62, respectively, forming two forced circulating loops between the two vapor tanks and the two groups of heat-exchange tube, respectively. The pumps can increase the flow rate of the circulated cooling agent to improve the heat exchange effect; and
(b) each group of the heat-exchange tube is composed of a plurality of straight tubes with headers at both ends thereof.

In Fig. 2, there are six (6) groups of heat exchange tube, each of which has two headers at opposite sides thereof. The header at the left side is connected to the inlet pipes or outlet pipes, respectively, as that in Fig. 1, and the adjacent headers 701 and 702 at the right side are connected via a communicating pipe 801. The group of heat-exchange tube 501 consisting of two bundles of tube, the vapor tank 61, the pump 81 and relevant pipes form a first circulating loop. The adjacent headers 703 and 704 as well as 705 and 706 at the right side are connected via communicating pipes 802 and 803, respectively, and the headers 604 and 605 at the left side are connected via a communicating pipe 800, the heat-exchange tube group 502 consisting of four bundles of tube, the vapor tank 62, the pump 82 and relevant pipes form a second circulating loop. Each of the heat-exchange tube group 501 or 502 can comprise another 2, 4, 6...... bundles of tube depending on the requirement, and each pair of adjacent headers can be connected via communicating pipes 804, 805......correspondingly.

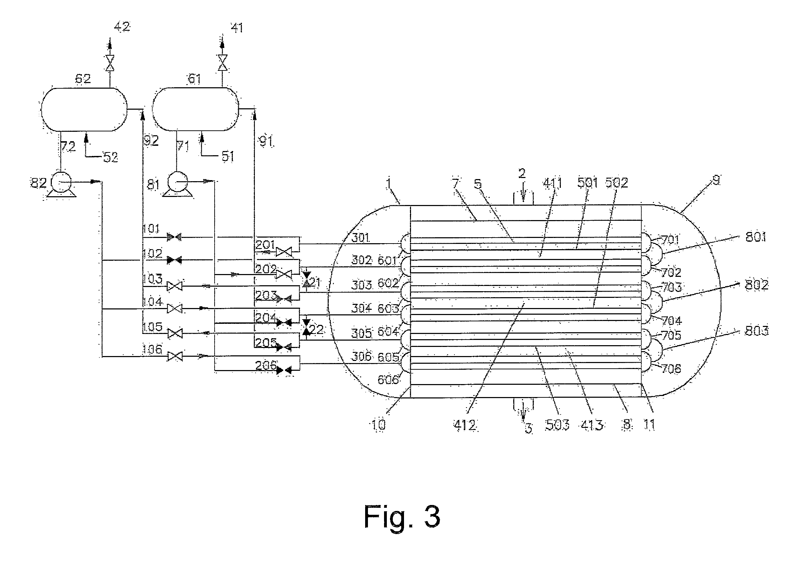

Fig. 3 shows a composite reaction apparatus comprising two vapor tanks and a horizontal reaction device, which apparatus comprises a horizontal reaction device 9 and two vapor tanks 61, 62. Said vapor tanks 61, 62 have pipes 51, 52 for supplementing water and vapor outlet pipes 41, 42 having vapor adjusting valves, respectively. The reaction device 9 comprises a shell 1, a reaction gas inlet 2 and an outlet 3 on the shell 1, a porous gas distributor 7 on the top of the shell 1, a porous gas collection plate 8 at the bottom thereof, two partition plates 10 and 11 in the shell 1, a catalyst layer 4 disposed between the partition plates and several groups of heat-exchange tube 501, 502, 503 disposed horizontally within the catalyst layer 4. There are headers 601, 602......606 at the left side of the heat-exchange tube groups 501, 502, 503, and headers 701, 702......706 at the right side thereof. The right headers 701 and 702, 703 and 704, 705 and 706 are connected via communicating pipes 801, 802, 803, respectively, to form three groups of heat-exchange tube 501, 502, 503. The headers 602, 604, 606 at the left side are connected to inlet pipes 302, 304, 306, respectively, and headers 601, 603, 605 are connected to outlet pipes 301, 303, 305, respectively. The vapor tanks 61, 62 are connected to the inlet pipes 302, 304, 306 of the groups of heat-exchange tube 501, 502, 503 via water pipes 71, 72, pumps 81, 82, water pipes 102, 104, 106 having valves and water pipes 202, 204, 206 having valves, respectively. The outlet pipes 301, 303, 305 of the groups of heat-exchange tube 501, 502, 503 are connected to vapor-water pipes 201, 203, 205 having valves and vapor-water pipes 101, 103, 105 having valves, respectively, and, in turn, connected to the vapor tanks 61 or 62 via vapor-water pipes 91 or 92.

In the apparatus shown in Fig. 3, the groups of heat-exchange tube 501, 502, 503 may optionally be connected in series with each other. For example, the outlet pipe 303 of the group of heat-exchange tube 502 can be connected to the inlet pipe 302 of the group of heat-exchange tube 501 via a communicating pipe 21 having a valve. By opening the valve in the communicating pipe 21, the valve in the water pipe 104 and the valve in the vapor-water pipe 101, or opening the valve in the water pipe 204 and the valve in the vapor-water pipe 201 while closing the valves in the vapor-water pipes 103, 203 and the valves in the water pipes 102, 202, a circulating loop consisting of the two groups of heat-exchange tube 501 and 502 connected in series and the vapor tank 61 or 62 can be formed.

Alternatively, the outlet pipe 305 of the group of heat-exchange tube 503 and the inlet pipe 304 of the group of heat-exchange tube 502 can be connected via a communicating pipe 22 having a valve, by opening the valve in the communicating pipe 22, the valve in the water pipe 106 and the valve in the vapor-water pipe 103 or opening the valve in the water pipe 206 and the valve in the vapor-water pipe 203 while closing the valves in the vapor-water pipes 105, 205 and the valves in the water pipes 104, 204, a circulating loop consisting of the two groups of heat-exchange tube 502, 503 connected in series and vapor tank 61 or 62 can be formed.

In addition, the three groups of heat-exchange tube 501, 502, 503 in figure 3 may be connected in series by adjusting the valves in the communicating pipes 21, 22 and the valves in the water pipe 106 and the vapor-water pipe 101 connected to the vapor tank 61, or the valves in the water pipe 206 and the vapor-water pipe 201 connected to the vapor tank 62. That is, by opening the valves in the communicating pipes 21, 22 connecting the inlet and outlet pipes of the groups of heat-exchange tube 501, 502, 503 while closing the valves in the water pipes 102, 104, 202, 204 and the vapor-water pipes 103, 105, 203, 205 connecting the inlet and outlet pipes to the vapor tanks 61, 62, a circulating loop can be formed by the three groups of heat-exchange tube connected in series. Such a circulating loop connected in series may be used in the case when the temperature difference between the front and end parts of the catalyst layer is not significant.

In the circulating loops wherein two or three groups of heat-exchange tube are connected in series, or two groups of heat-exchange tube are communicated to the same one vapor tank concurrently, the vaporization temperature of the cooling agent is identical in all heat-exchange tubes except for the part adjacent to the water inlet pipes, such a technical effect together with the high flow rate in the heat-exchange tubes connected in series facilitate the increase of the heat transfer coefficient.

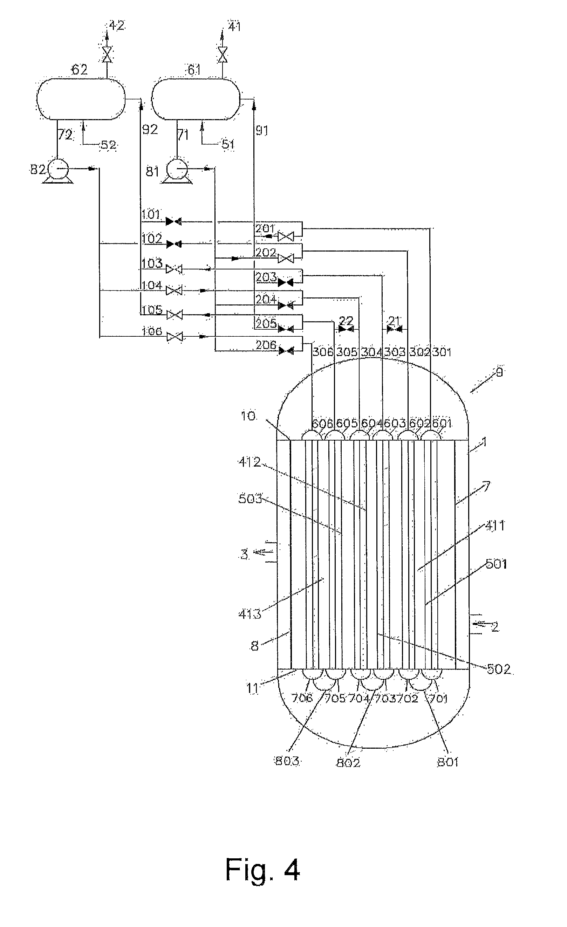

Fig. 4 shows an apparatus comprising a vertical water-cooling reaction device and two vapor tanks. The structures that are similar to those shown in Fig. 3 will not be described repeatedly. The only difference in the apparatus of figure 4 is the reaction device being vertical while that in Fig. 3 being horizontal.

In Fig. 4, the shell 1 and the groups of heat-exchange tube 501, 502, 503 are disposed vertically, allowing the cooling agent to flow in the tubes vertically, and two partition plates 10, 11 are disposed at the top and the bottom of the catalyst layer 4 outside the groups of heat-exchange tube, forming a heat-exchange reaction zone by the shell 1, an upper partition plate 10, a lower partition plate 11, the porous gas distributor 7 at one side of the catalyst layer 4 and the porous gas collection plate 8 at another side thereof.

During operation, the reaction gas undergoes reaction while flowing horizontally in the catalytic reaction zones 411, 412, 413, and exchanges heat with the cooling agent flowing vertically in the groups of heat-exchange tube 501, 502, 503. In the apparatus shown in Fig. 4, the groups of heat-exchange tube are supported on a bottom cap of the shell via a supporting plate (which is not shown). Ceramic pellets may be used to fill in the volume between the bottom cap and the groups of heat-exchange tube.

In the reaction devices shown in Fig. 1 to Fig. 4, the number of the groups of heat-exchange tube can be increased as desired, and the header for connecting each pair of adjacent groups of heat-exchange tube can be connected via additional communicating pipe correspondingly, such as communicating pipe 804, 805.......

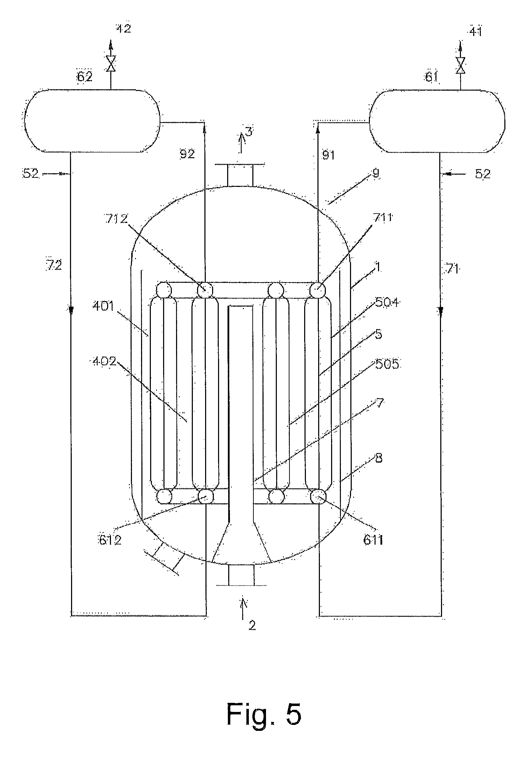

Fig. 5 shows an apparatus comprising a radial reaction device, which reaction device comprises a plurality of groups of heat-exchange water tube. The reaction device shown in the figure comprises reactor shell 1, groups of the heat-exchange tube 5 disposed vertically, porous gas collection plate 8 and central porous gas distributor 7.

Said groups of heat-exchange tubes 5 consist of an outer heat-exchange tube group and an inner heat-exchange tube group.

Said outer heat-exchange tube group comprises an upper outer current-collecting ring 711, a lower outer current-dividing ring 611 and a group of heat exchange tube 504 fluidly connecting the upper outer current-collecting ring 711 and lower outer current-dividing ring 611 together.

Said inner heat-exchange tube group comprises an upper inner current-collecting ring 712, a lower inner current-dividing ring 612 and a group of heat exchange tube 505 fluidly connecting the upper inner current-collecting ring 712 and lower inner current-dividing ring 612 together. Said outer and inner heat-exchange tube groups have different radii but are disposed concentrically.

There are catalyst layers 401, 402 in the shell 1 between the porous gas collection plate 8 and the central porous gas distributor 7 outside the outer and inner heat-exchange tube groups. The lower current-dividing ring 611 is connected to the vapor tank 61 via a water pipe 71, and the upper current-collecting ring 711 is connected to the vapor tank 61 via a vapor-water pipe 91, forming a circulating loop of the outer heat-exchange tube group, while the lower current-dividing ring 612 is connected to the vapor tank 62 via a water pipe 72, and the upper current-collecting ring 712 is connected to the vapor tank 62 via a vapor-water pipe 92, forming a circulating loop of the outer heat-exchange tube group.

During operation, the reaction gas enters the reaction device 9 from the inlet 2, passes through the porous gas distributor 7, flows radially in the catalyst layers 401, 402 outside the groups of heat-exchange tube 504, 505 and undergoes reaction and heat-exchange, passes through the porous gas collection plate 8 and exits the reaction device from the outlet 3. Alternatively, the functionalities of the inlet 2 and the outlet 3 may be exchanged so that the gas flows inwardly in the catalyst layers 401, 402 and undergoes reaction and heat-exchange. Again, the heat-exchange tube groups are supported on a bottom cap of the shell by a supporting plate (which is not shown).

Alternately, said outer heat-exchange tube group comprising the upper outer current-collecting ring, lower outer current-dividing ring and a group of the heat-exchange tube 504 can be composed of a plurality of sub-outer heat-exchange tube groups, each of the sub-outer heat-exchange tube group consists of a sub-group of outer heat-exchange tube, a sub-upper outer current-collecting ring and a sub-lower outer current-dividing ring, all the sub-upper outer current-collecting rings and sub-lower outer cunent-dividing rings are disposed concentrically and fluidly communicated, respectively, and connected to the same one vapor tank; while said inner heat-exchange tube group comprising the upper inner current-collecting ring, lower inner current-dividing ring and a group of heat-exchange tube 505 can be composed of a plurality of sub-inner heat-exchange tube groups, each of the sub-inner heat-exchange tube group consists of a sub-upper inner current-collecting ring, a sub-lower inner current-dividing ring and a sub-group of heat-exchange tube 505, all the sub-upper inner current-collecting rings and sub-lower inner current-dividing rings are disposed concentrically and fluidly communicated, respectively, and connected to the same one vapor tank.

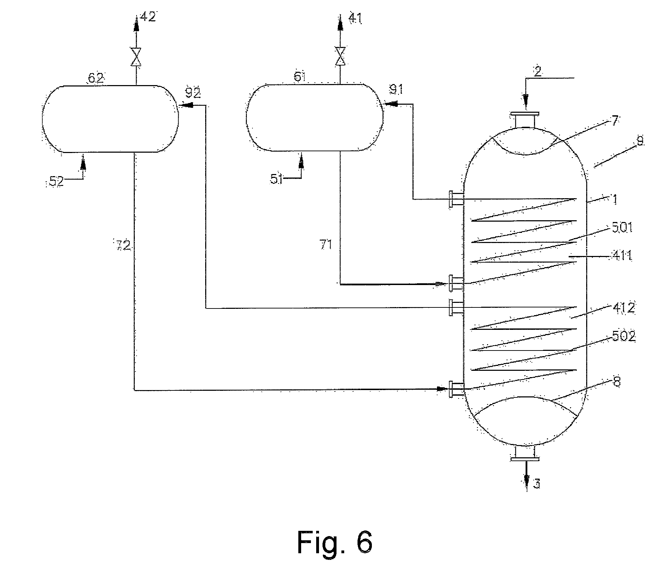

Fig. 6 shows an apparatus comprising an axial reaction device having a plurality of heat-exchange tube groups. The reaction device disposed vertically comprises shell 1, the catalyst layer 4 in the shell 1 is divided into a plurality of catalytic reaction zones 411, 412, at least two of the catalytic reaction zones contain groups of heat-exchange tubes 501, 502, respectively, each group of the heat-exchange tube comprises straight or helix tubes with cooling agent therein and is connected via a water pipe and a vapor-water pipe to the vapor tank 61 or 62 having different vaporization pressures.

### One-step Synthesis of Dimethyl Ether

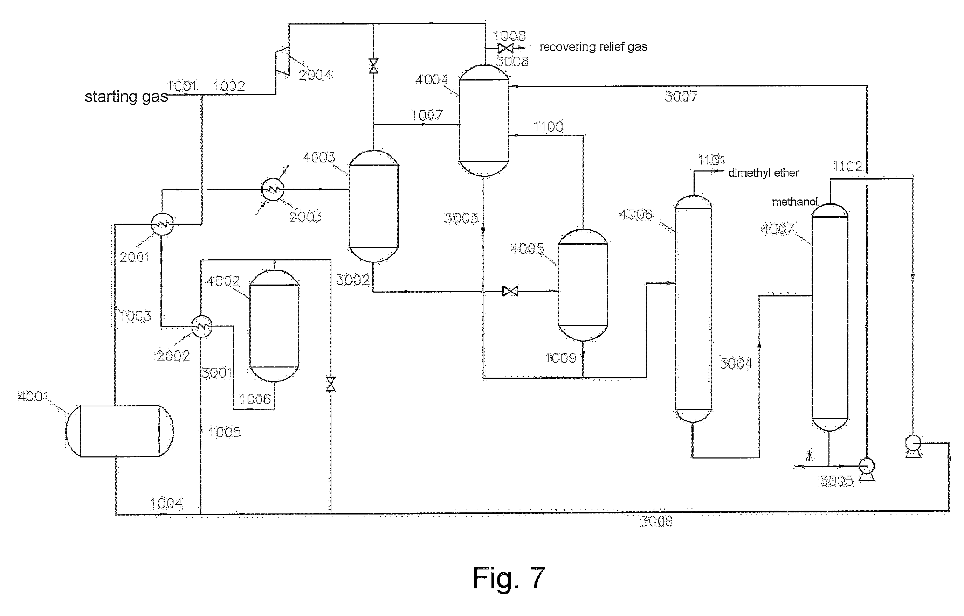

Fig. 7 is a schematic flow chart of the one-step synthesis of dimethyl ether. A starting gas 1001, comprising H₂, CO, CO₂ and a small amount of CH₄ and N₂, etc. and having a hydrogen/carbon ratio of 0.7-2.5 and a pressure of 5-12MPa, may be a syngas obtained by gasification of coal water slurry or by gasification of powdered coal with pure oxygen, or may be obtained by transformation of natural gas. The starting gas 1001 combines with the recycle gas 1002 from a cycle machine 2004 to form gas 1003. After heated to about 210°C by a gas-gas heat exchanger 2001, the gas 1003 enters a methanol synthesis reactor 4001 consisting of the apparatus of the invention to proceed a reaction for the synthesis of methanol. The methanol synthesis reactor 4001 has at least two (2) groups of heat-exchange tubes whose vaporization pressure and temperature can be adjusted independently. The apparatus of the present invention can clear away the restriction on heat removal encountered by the prior art water-cooling reactor, greatly reducing the recycle ratio of methanol synthesis, such that the syngas 1004 exiting the methanol synthesis reactor 4001 comprises more than 10% by volume of methanol. The syngas 1004 combines with part of the recovered methanol coming from a rectifying tower via a pipe 3006 to form syngas 1005 which passes a gas-gas heat exchanger 2002 and combines with the other part of the recovered methanol coming from the rectifying tower before entering a dehydration reactor 4002. The other part of the recovered methanol coming from the rectifying tower or the mixture of the other part of the recovered methanol and the syngas 1004 has a cold auxiliary line to adjust the temperature of the gas 1005 entering the dehydration reactor 4002. The temperature resulted from the adjustment depends on the specific catalyst. For example, the temperature is adjusted to about 200 °C when a molecular seive is used as the catalyst; and to about 300°C whenγ -alumina is used as the catalyst. The gas 1005 entering the tower undergoes methanol dehydration on the methanol dehydration catalyst. Outlet reaction gas 1006 exiting the tower comprises more than 5% of dimethyl ether. After passing a pipe 3001 and two gas-gas heat exchangers 2002, 2001, the temperature of the gas 1006 is decreased to about 100°C. The gas 1006 is further cooled to lower than 40°C by a water cooler 2003, and then separated by a gas-liquid separator 4003 to give condensed methanol, dimethyl ether and water. Gas 1007 enters an absorption towers 4004 to allow more products such as methanol, dimethyl ether, etc. to be absorbed. Alternatively, except for a small amount of relief gas 1008 which is sent to the absorption tower 4004, the remaining gas exiting the separator 4003 is used as a recycle gas 1002, after passing the cycle machine 2004, to combine with fresh starting gas 1001 to synthesize methanol, dimethyl ether. The liquid phase exiting the bottom of the separator 4003 passes a pipe 3002 and enters a reduced pressure flash drum 4005 to obtain a pressure of about 2 MPa. A flash gas 1100 is formed by H₂, CO, CO₂, etc. having a low solubility and enters the absorption tower 4004 after combining with the relief gas 1007. After combining with the gas from the

separator 4003, the resultant gas is absorbed by water or liquid methanol from the methanol recovery tower 4007 via a pipe 3007. Part of the gas exiting the absorption tower 4004 is recycled or leaves the system via a relief pipe 3008. Liquid methanol and dimethyl ether from the separator 4003 and the absorption towers 4004 passes a pipe 3003 and combines with the liquid methanol and ether from the flash drum 4005. The resultant liquid is sent to a rectifying tower 4006 to give product dimethyl ether 1101 by fractionation. The aqueous solution of methanol exiting the bottom of the rectifying tower 4006 is sent to the methanol recovery tower 4007 via a pipe 3004 to be fractionated. Process water is discharged from the bottom of the recovery tower 4007 via a pipe 3005, part of which is pressurized by a pump and sent to the absorption tower 4004. Methanol 1102 recovered from the top of the recovery tower 4007 is sent via a pipe 3006 to the methanol dehydration tower 4002 to be dehydrated into dimethyl ether, or sold as product methanol.

### Synthesis of Hydrocarbons from Syngas via Methanol

The synthesis of hydrocarbons from syngas via methanol involves the following principal reactions:
1. Synthesis of methanol:

   CO + 2H₂ → CH₃OH + 102.5 KJ (1)
2. Dehydration of methanol to produce hydrocarbons:

   CH₃OH → H₂O + -CH₂- +44.73KJ or (2)

   dehydration of methanol to produce dimethyl ether, followed by dehydration of dimethyl ether to produce hydrocarbons:

   CH₃OH → 1/2(CH₃)₂O + 1/2H₂O + 10.08 KJ (3)

   1/2 (CH₃)₂O → -CH₂- + 1/2H₂O + 34.65 KJ (4)

The product hydrocarbons include alkanes, alkenes (e.g. ethylene, propylene, etc.).

It can be seen that all of the above reactions are highly exothermic. If reaction heat is not removed in time, catalyst will be deactivated by excessively high temperature. According to recent literature data, the content of dimethyl ether in the reaction gas at the outlet of the reactor is less than 4% when a tube-shell water-cooling fixed bed reactor is used in one-step synthesis of dimethyl ether.

When the apparatus of the invention is used to synthesize methanol and dimethyl ether, the restriction on heat removal in current technology is broken. According to the invention, recycle ratio may be reduced to less than 2 from 5-10, and the content of methanol in the reaction gas of methanol synthesis may be promoted to 10%-60% from 3%-6%. The reaction gas with high content of methanol or methanol and dimethyl ether thus obtained in methanol synthesis may be sent to a dehydration reactor to produce hydrocarbons without cooling and separation. The reactions involved are also highly exothermic, and a multistage water-cooling reactor may be used. A molecular seive such as ZSM-5 is used as the catalyst. The reaction temperature is 260-420 °C, most preferably 280-380°C. Reaction heat is removed by boiling cooling water to co-produce steam of about 10MPa.

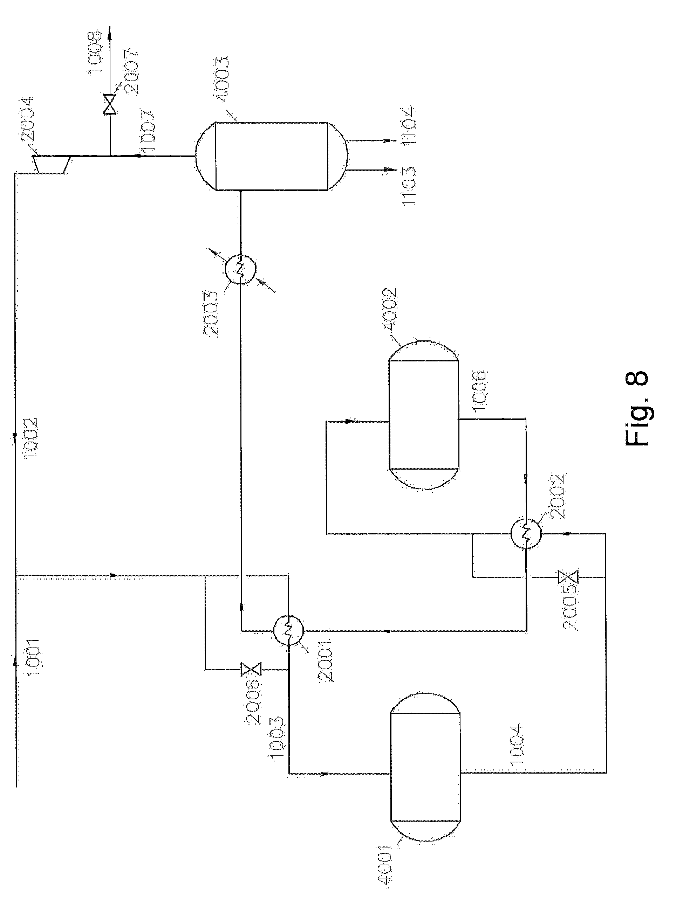

Fig. 8 is a schematic flow chart of the one-step synthesis of hydrocarbons from syngas via methanol. A starting gas 1001, comprising H₂, CO, CO₂ and a small amount of CH₄, N₂, etc., having a hydrogen/carbon ratio of 0.7-2.5 and a pressure of 2-15MPa, may be syngas obtained by gasification of coal water slurry or by gasification of powdered coal with pure or rich oxygen, or may be obtained by transformation of natural gas. The starting gas 1001 combines with the recycle gas 1002 from a cycle machine 2004 to form gas 1003. After heated to about 210°C by a gas-gas heat exchanger 2001, the gas 1003 enters a methanol synthesis reactor 4001 consisting of the apparatus of the invention to react. The methanol synthesis reactor 4001 has at least 2 groups of heat-exchange tubes capable of adjusting vaporization pressure and temperature independently. Syngas 1004 exiting the methanol synthesis reactor 4001 comprises more than 10% by volume of methanol. The syngas 1004 exchanges heat with gas 1006 exiting a methanol dehydration reactor 4002 at a gas-gas heat exchanger 2002 to obtain a temperature of about 300 °C before entering the methanol dehydration reactor 4002 to undergo methanol dehydration on a methanol dehydration catalyst. The gas 1006 exiting the reactor 4002 is cooled to about 100°C by two gas-gas heat exchangers 2002, 2001, further cooled to about 40°C by a water cooler 2003, and then separated by a gas-liquid separator 4003 to give condensed hydrocarbons 1103 and water 1104 which are discharged as liquid phase. Gas 1007, except for a small amount of relief gas 1008 which is released via a purge valve 2007 for other purpose, is used as a recycle gas 1002, after passing the cycle machine 2004, to combine with fresh starting gas 1001 to synthesize methanol. Reference numbers 2006 and 2005 in Fig. 8 represent bypass valves for regulating temperature of the gases entering reactors 4001 and 4002 respectively.

The cycle machine 2004 may be obviated in Fig. 8. Thus, the syngas goes through the methanol synthesis reactor and the methanol dehydration reactor without recycling. The gas 1007 exiting the gas-liquid separator 4003 is sent totally as the relief gas 1008 to Integrated Gasification Combined Cycle (IGCC), i.e. gas/steam integrated recycle generator. The starting gas 1001 is the gas entering the synthesis tower, which is heated to about 210°C by the gas-gas heat exchanger 2001 before entering the methanol synthesis reactor 4001 to undergo methanol synthesis.

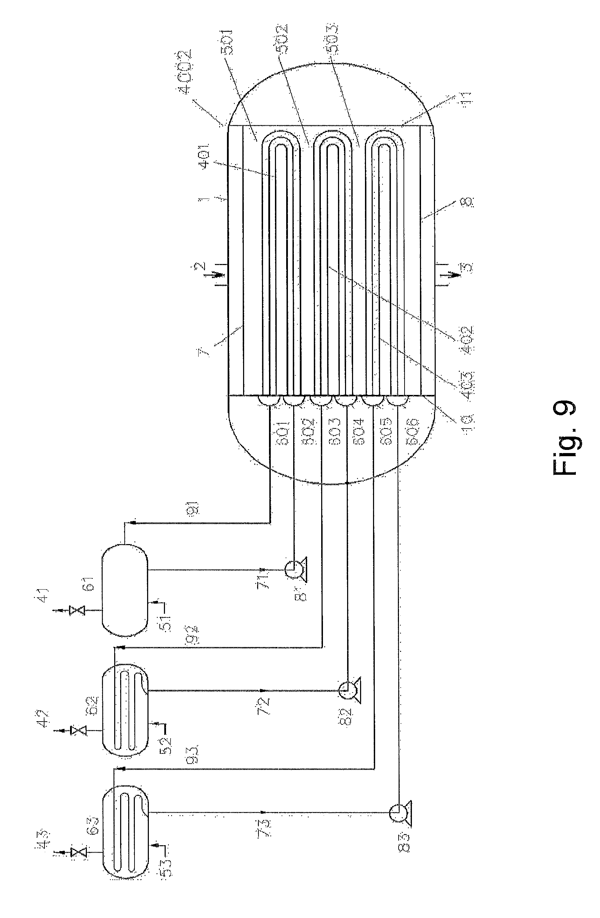

Fig. 9 is a schematic drawing of a reactor containing three groups of heat-exchange tubes connected to vapor tanks in the same shell. As schematically shown in the figure, methanol dehydration catalyst is loaded in a plurality of layers within a reactor. The apparatus shown is a horizontal composite reaction device comprising a plurality of U-shaped tubes with three vapor tanks. The apparatus is composed of a methanol dehydration reactor 4002 and vapor tanks 61, 62, 63 connected thereto. The tanks 61, 62, 63 are communicated with vapor outlet pipes 41, 42, 43 having vapor adjusting valves and pipes 51, 52, 53 for adding water. The methanol dehydration reactor 4002 is composed of a shell 1, a reaction gas inlet 2 and outlet 3 on the shell 1, a porous gas distributor 7 on the top of the shell 1 and a porous gas collection plate 8 at the bottom, partition boards 10, 11 at two opposite sides in the shell 1 with three catalyst layers 401, 402, 403 disposed therebetween, and three groups of U-shaped heat-exchange tubes 501, 502, 503 in the catalyst layers 401, 402, 403 for removing heat with cooling medium. The groups of heat-exchange tubes 501, 502, 503 are composed of U-shaped tubes, the open ends of which are communicated with headers 601, 602 ...... 606. The headers 602, 604, 606 are connected to the vapor tanks 61, 62, 63 via cooling agent inlet pipes 71, 72, 73, and the headers 601, 603, 605 are connected to the vapor tanks 61, 62, 63 via cooling agent inlet pipes 91, 92, 93, so as to form circulating loops of cooling agent between the three groups of heat-exchange tubes 501, 502, 503 and the three vapor tanks 61, 62, 63. If low cooling agent temperature is needed ( < 300 °C), water may be used in the heat-exchange tube groups such as the heat-exchange tube group 501 connected to the vapor tank 61 as shown in the figure. If higher cooling agent temperature is needed (300-350°C), it is more desirable to remove heat with heat conducting oil or molten salts. For example, heat conducting oil is used in the heat-exchange tube groups 502, 503 connected to the vapor tanks 62, 63 as shown in the figure to carry heat to heat exchangers in the vapor tanks 62, 63 and transfer the heat to water there to produce steam. In the catalyst layer 401, γ -alumina is disposed as the catalyst for dehydration of methanol to produce dimethyl ether. A molecular seive catalyst such as ZSM-5 is disposed in the catalyst layers 402, 403 for dehydration of methanol. The heat-exchange tube groups 501, 502, 503 may also be composed of straight tubes with headers at both ends like the case shown in Fig. 3. When necessary, an adiabatic catalyst layer may be disposed between the bottom of the heat-exchange tube group 503 and the porous gas collection plate 8.

### Preparation of Hydrocarbons by Fischer-Tropsch Reaction from Syngas

According to the invention, there is provided an energy-saving method for preparation of hydrocarbons by Fischer-Tropsch reaction from syngas at low recycle ratio and high CO conversion, and a corresponding apparatus.

Fischer-Tropsch reaction is generally used to produce liquid hydrocarbons from CO and H₂ in the presence of a catalyst. Preparation of hydrocarbons by Fischer-Tropsch reaction from syngas involves the following principal reactions:

CO + 2H₂ → (-CH₂-) + H₂O + 165 KJ (1)

2CO + H₂ → (-CH₂-) + CO₂ + 204.8 KJ (2)

Conventional catalysts include cobalt-based or iron-based catalysts. Iron-based catalysts include precipitated iron and molten iron.

There are two problems concerning Fischer-Tropsch synthesis. First, despite the simple starting material mainly composed of CO and H₂, the product hydrocarbons of Fischer-Tropsch reaction are very complex as the number of carbons varies widely, including alkanes, alkenes, cycloalkanes, aromatics, etc.. The resultant heavy hydrocarbons may condense and deposit on the catalyst surface, which will impact the catalytic reaction. Secondly, since the products of Fischer-Tropsch reaction have a wide range of molecular weights, the product hydrocarbons with wax as the main component have to be modified by hydrogenation cracking and hydrogenation isomerization to produce liquid products such as gasoline. According to an existing process as proposed by Sasol, of the product hydrocarbons generally produced by Fischer-Tropsch reaction, wax and the like are separated from the bottom of the Fischer-Tropsch reactor, hot condensate is separated from the bottom of the heat exchanger, and cold condensate is separated from the gas using a separator with a water cooler. Then, these products separated from the synthetic reaction gas are sent to a reactor for modifying the hydrocarbons and then separated. This process suffers from a long flow line, plenty of equipments and large investment. Further more, condensation, separation, reheating, hydrogenation and modification of the products lead to increase of energy consumption.

Meanwhile, all of the above reactions involved in Fischer-Tropsch synthesis are highly exothermic. If reaction heat is not removed in time, catalyst will be deactivated by excessively high temperature. Catalytic reaction does not proceed at the same rate on different parts of the catalyst. Generally, since reaction at the front part of a reactor is relatively far from equilibrium, its rate is faster and more reaction heat is released. In contrast, reaction at the back of the reactor approaches equilibrium, resulting in a lower reaction rate and less reaction heat that is released. As mentioned above, the cooling agent in an existing tube-shell water-cooling reactor has an even temperature along its length. If the temperature of the cooling agent is decreased to enlarge the temperature difference for heat-exchange and speed up heat removal so as to meet the requirement of the upper or front part of the reactor on high reaction rate and intensive reaction heat, the reaction heat at the lower or back part of the reactor will be decreased and thus the heat removed will be more than the reaction heat, leading to decrease of the temperature thereat. Correspondingly, the reaction rate here is lowered, and the reaction will stop when the catalyst loses its activity due to too low a temperature. Thus, it is difficult to allow reaction to proceed at optimal temperature along the whole length of the reactor. As such, recycle gas that is 2-4 times as much as starting gas is used in current technology to remove reaction heat from the catalyst layer. In order to solve this problem, a cooling agent with varying temperatures at different zones of a reactor is used in the invention rather than using a cooling agent of the same temperature, so that the heat exchange in the reaction can be designed according to the amount of reaction heat to be removed. Specifically, the reactor can be divided into several zones along the flowing direction of the reaction gas in the catalyst layer, and exchange heat with the cooling agent through heat-exchange tubes in various zones. For such reactions as those in Fischer-Tropsch synthesis at 200-300 °C, pressurized heated water may be used as the heat carrier, for evaporation heat of the liquid in the reactor is much higher than the sensible heat absorbed by the cooling agent due to rise of temperature. For even higher reaction temperature, mineral oil, heat conducting oil or molten salts having low volatility may be used as the cooling agent to carry heat to a vapor tank through a circulating loop to be recovered by producing steam. When water is used as the cooling agent, the heat carrier removes the Fischer-Tropsch reaction heat, and steam is co-produced directly. Fischer-Tropsch synthesis catalyst may be disposed in the heat-exchange tubes, like the case of Lurgi tube-shell reactor. Here, the heat-exchange tubes act as the reaction tubes, and boiler water covers the shell distance outside the reaction tubes. Alternatively, catalyst may be disposed outside the heat-exchange tubes. In such a case, the heat-exchange tubes are the water pipes, resulting in a water pipe reactor. The water pipe may be straight or helical. The water pipe may be positioned horizontally, resulting in a horizontal water-cooling methanol tower. The first advantage of this method lies in the fact that heat-exchange tubes are divided into several groups in the catalyst zone. The groups of heat-exchange tubes may be communicated with different vapor tanks having different vaporization pressures and temperatures. For example, at earlier stage of the reaction, vaporization in the front heat-exchange tubes occurs at low pressure and temperature to enlarge the temperature difference, so that heat transfer is enhanced. Thus, the problem that the reaction is too fast and the reaction heat is too much in the front of the reactor is solved. The catalytic reaction rate and the reaction heat are reduced at the back of the reactor, and the vaporization pressure and temperature are increased in the heat-exchange tubes correspondingly to reduce the temperature difference, so as to prevent the reaction temperature from getting too low due to decrease of reaction heat and excessive removal of heat. The second advantage of the invention lies in the fact that the pressure in the vapor tank is adjustable. Along with the use of the catalyst, the catalytic reaction proceeds to the back of the reactor. When the reaction heat at the back increases, the vaporization pressure of the heat-exchange tubes in the back part of the catalyst may be lowered, or the heat-exchange tubes may be connected to the vapor tank with lower pressure, so that vaporization in the water pipes occurs at low temperature and pressure at later stage of the reaction, and the temperature difference and the heat-transfer rate are thus increased. Pressure in the foregoing heat-exchange tubes and pressure adjusting vapor tanks may be selected to be 0.5-16MPa, as desired, and pressure difference between the heat-exchange tubes and the pressure adjusting vapor tanks connected thereto is 0.1-15MPa.

Since a multistage water-cooling reactor is used in Fischer-Tropsch synthesis, the recycle ratio may be reduced to less than 1 from 2-5, and the content of hydrocarbons in the Fischer-Tropsch reaction gas is thus increased greatly. According to the invention, Fischer-Tropsch synthesis may be combined with hydrogenation for modifying the resultant hydrocarbons in two ways. One way is to connect a hydrogenation reactor in tandem form to the Fischer-Tropsch reactor from behind. The hydrocarbons produced by Fischer-Tropsch reaction are sent to the hydrogenation reactor for modification without isolating the hydrocarbons from the gas phase beforehand. The selection of hydrogenation temperature depends on Fischer-Tropsch reaction and hydrogenation catalyst. For example, when Fischer-Tropsch reaction is carried out at 200-300°C, hydrogenation may be carried out at 260-380°C. The other way is to blend a Fischer-Tropsch catalyst and a hydrogenation catalyst such as ZSM-5 together, and then load the resultant Fischer-Tropsch composite catalyst in a synthesis tower. For example, an Fe (iron), Fe-Mn (iron-manganese) or Co (cobalt) based catalyst may be blended with ZSM-5 to form a composite catalyst for use in a reaction at 230-260°C. As a result, gasoline fraction (C₅-C₁₁) is increased greatly, so that the quality of gasoline is improved. In such a way, the high content of hydrocarbons obtained by Fischer-Tropsch synthesis needn't be isolated from the gas phase and may be modified directly in a hydrogenation reactor to produce final products such as gasoline. Co, Fe, Fe-Mn family catalysts and ZSM-5 hydrogenation catalyst may be mixed evenly before loading, or loaded alternately from the top down.

The problem of large recycle ratio and low product concentration suffered by a fixed bed reactor for Fischer-Tropsch synthesis is solved by the invention. For this purpose, a Fischer-Tropsch reactor having a plurality of heat-exchange water tube groups capable of adjusting vaporization pressure and temperature independently is used, wherein the temperature difference for heat-exchange and the heat removal are promoted in the zones receiving more reaction heat by decreasing the vaporization temperature and pressure in the tubes therein, while the temperature difference for heat-exchange in the zones receiving less reaction heat is maintained small to prevent the temperature from getting too low.

Fig. 8 may also be seen as a schematic flow chart for synthesis of hydrocarbons by Fischer-Tropsch reaction from syngas. The difference is that starting gas 1001 combines with the recycle gas 1002 from a cycle machine 2004 to form gas 1003; after heated to about 200°C by a gas-gas heat exchanger 2001, the gas 1003 enters a Fischer-Tropsch synthesis reactor 4001 to react; syngas 1004 exiting the Fischer-Tropsch synthesis reactor 4001 exchanges heat with gas 1006 exiting a hydrocarbon modification reactor 4002 at a gas-gas heat exchanger 2002 to obtain a temperature of about 300 °C before entering the hydrocarbon modification reactor 4002 to undergo hydrocarbon modification on a molecular seive catalyst.

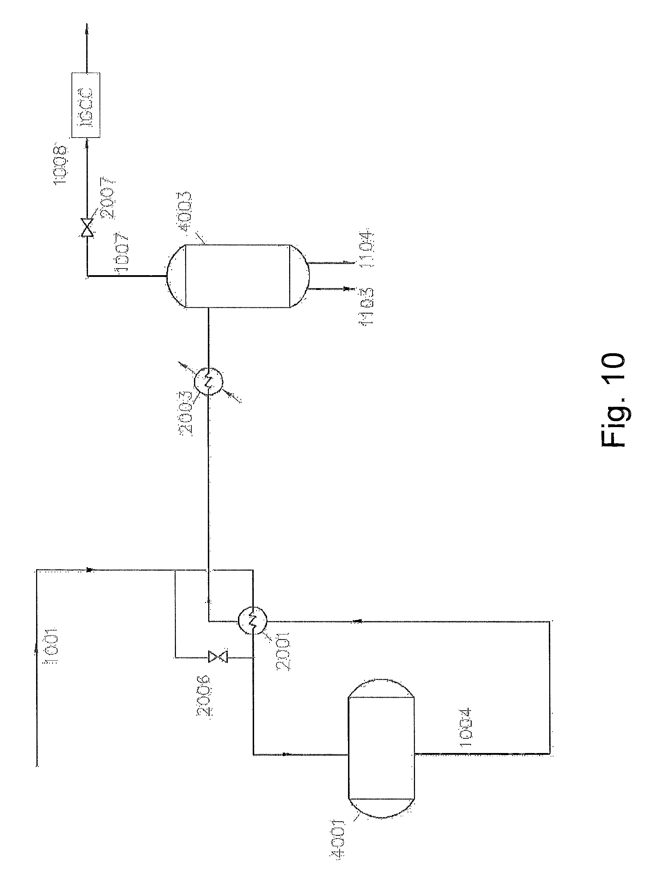

Fig. 10 is a schematic flow chart for a composite Fischer-Tropsch reaction. The difference from Fig. 8 is that only a Fischer-Tropsch synthesis reactor 4001 is used here. Syngas 1001, used as the gas entering the synthesis tower without recycling, is heated to about 250°C by a gas-gas heat exchanger 2001 before entering the Fischer-Tropsch synthesis reactor 4001 to undergo Fischer-Tropsch reaction. In the Fischer-Tropsch synthesis reactor 4001 is loaded a mixture of a transition metal catalyst such as Fe, Mn, Co, etc., or an Fe-Mn ultrafine particulate catalyst and a molecular seive such as ZSM-5, etc.. The syngas 1001 undergoes Fischer-Tropsch synthesis in the Fischer-Tropsch synthesis reactor 4001. In the same reactor 4001, the resultant hydrocarbons to be modified further react with hydrogen in the gas in the form of hydrogenation cracking, hydrogenation isomerization, etc.. The gas exiting the Fischer-Tropsch synthesis reactor 4001 is cooled by a heat exchanger 2002 and a water cooler 2003, and then separated by a separator 4003.

### Preparation of Natural Gas by Methanation of Syngas

Methanation of H₂, CO, CO₂ in syngas or coke oven gas made by gasifying coal may be used to produce man-made natural gas. The reactions involved are highly exothermic, as shown below:

CO + 3H₂ = CH₄ + H₂O + 206KJ/mol (1)

CO₂ + 4H₂ = CH₄+2H₂O +165KJ/mol (2)

Since methanation reactions (1) and (2) are both highly exothermic, the temperature in the methanation reactor has to be controlled during the methanation of syngas to prevent the catalyst in the reactor from being overheated.

In the highly exothermic methanation reactions, when 1% CO is converted into methane, the temperature is increased by about 74°C; and when 1% CO₂ is converted into methane, the temperature is increased by about 60°C, both in adiabatic conditions. Obviously, in order to finish the methanation of high content of CO in syngas in the synthesis tower in just one pass, the intensive reaction heat has to be removed. Otherwise, the catalyst will be deactivated by the excessively high temperature. However, the reaction heat is determined by the reaction rate of the gas on the catalyst, wherein the reaction rate varies along the catalyst bed. For both axial towers and radial towers, the reaction rate climbs at the beginning, and a peak occurs in the front half part of the catalyst layer. Then, the reaction rate decreases gradually. In the back half part, the reaction slows down, and the reaction heat is reduced. For existing axial towers such as tube-shell water-cooling or gas-cooling heat-exchange reactors, the heat-exchange area is fixed from the top to the bottom of the catalyst layer, and the temperature of the cooling agent is also constant. Restricted by the temperature of the hot spot where the peak reaction rate occurs in the front half part of the catalyst layer as mentioned above, it is less likely to reduce greatly the recycle ratio of the synthesis tower having such a structure, and thus enhance the content of CO in the gas entering the synthesis tower to finish the synthesis in one pass.

The invention solves this problem in current technology by using a methanation tower having a plurality of heat-exchange water tube groups capable of adjusting vaporization pressure and temperature independently, wherein the temperature difference for heat-exchange and the heat removal are promoted in the zones receiving more reaction heat by decreasing the vaporization temperature and pressure in the tubes therein, while the temperature difference for heat-exchange in the zones receiving less reaction heat is maintained small to prevent the temperature from getting too low. In addition, a cross-flow water-cooling reaction device is applied, wherein the heat-exchange area is arranged along the flowing direction of the gas in the catalytic reaction of methanation according of the reaction rate and the reaction heat.

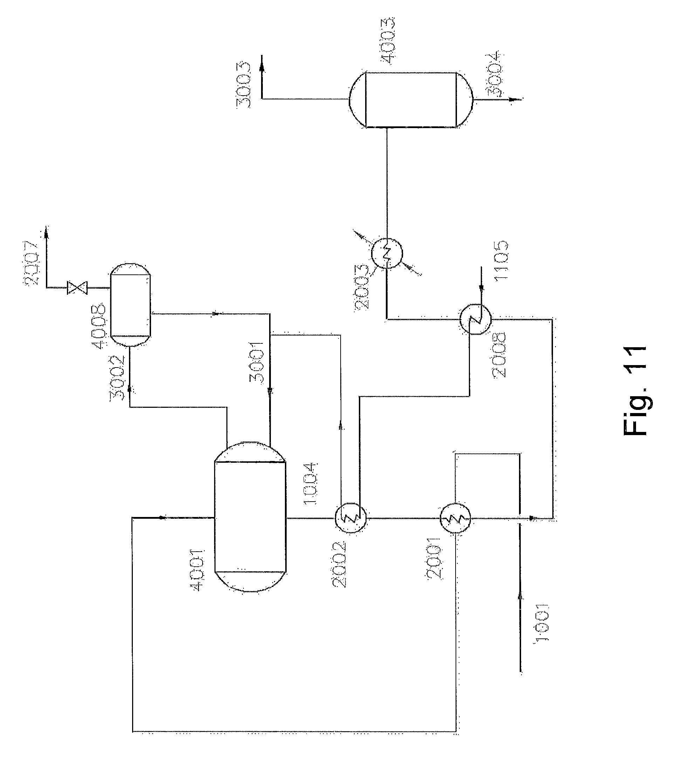

Fig. 11 is a schematic flow chart of the preparation of natural gas by methanation of syngas. A starting gas 1001, comprising H₂, CO, CO_{2,} CH₄ and a small amount of N₂, etc., having a hydrogen/carbon ratio of about 3 and a pressure of 1-10MPa, may be syngas or coke oven gas obtained by gasification of coal water slurry or by gasification of powdered coal with pure oxygen. The starting gas 1001 is heated to about 260°C by a gas-gas heat exchanger 2001, and then enters a methane synthesis reactor 4001 to undergo methanation, wherein CO and CO₂ in the syngas react with H₂ to produce methane. The methane synthesis reactor 4002 has at least 2 groups of heat-exchange tubes capable of adjusting vaporization pressure and temperature independently as shown in Fig. 1 and 2. The reaction gas 1004 containing about 1% by volume of CO leaves the methane synthesis reactor 4002 at about 350°C. The reaction gas 1004 is cooled to about 300°C by a heat exchanger 2002 which is used to heat boiler water, heats the gas 1001 entering the towers at the gas-gas heat exchanger, is cooled to about 100°C by a heat exchanger 2008 which is used to heat boiler water 1105, and further cooled to lower than 40°C by a water cooler 2003. The resultant is separated by a gas-liquid separator 4003 to give the product gas from a top pipe 3003 and water from a bottom pipe 3004. Boiler water is sent via a pipe 3001 to the methane synthesis reactor 4001 to absorb reaction heat. A mixture of vapor and water is sent via a pipe 3002 to a vapor tank 4008, from which steam is discharged via a top pipe 2007 with a valve.

### Method of Chemical-power co-production

The invention provides a method of chemical-power co-production. Syngas 004, obtained by gasifying a carbon-containing starting material 001 with water, oxygen, carbon dioxide and the like, is first subjected to chemical process 005 to produce chemical products. The unreacted syngas 007, as a fuel, is sent to an integrated recycle generator system combining electricity generation by gas 008 and by steam 009. The steam produced in gasification and purification of syngas and in chemical synthesis is also sent to the steam generator.

In an embodiment of the invention, the syngas produced by gasifying a carbon-containing starting material is subjected to no or small CO transformation, rendering the ratio of H₂/CO in the syngas, which is to be subjected to chemical synthesis, to be 0.3-2.2, more preferably 0.4-2.0.

In an embodiment of the invention, the syngas separated from the chemical products is totally sent to the gas/steam generator rather than recycled. Alternatively, a small part of the syngas is recycled, and the ratio of the recycle gas to the fresh syngas based on molar flow rate is less than 5. The chemical products obtained by the method of the invention include methanol, dimethyl ether and hydrocarbons. The exergy ratio of the chemical product to the power is 0.5-4.

In an embodiment of the invention, carbon dioxide is used to partly replace steam in the gasification of coal to produce syngas which is used as the starting material of the chemical-power co-production.

The fulfillment of this inventive method mainly relies on the use of the apparatus of the invention.

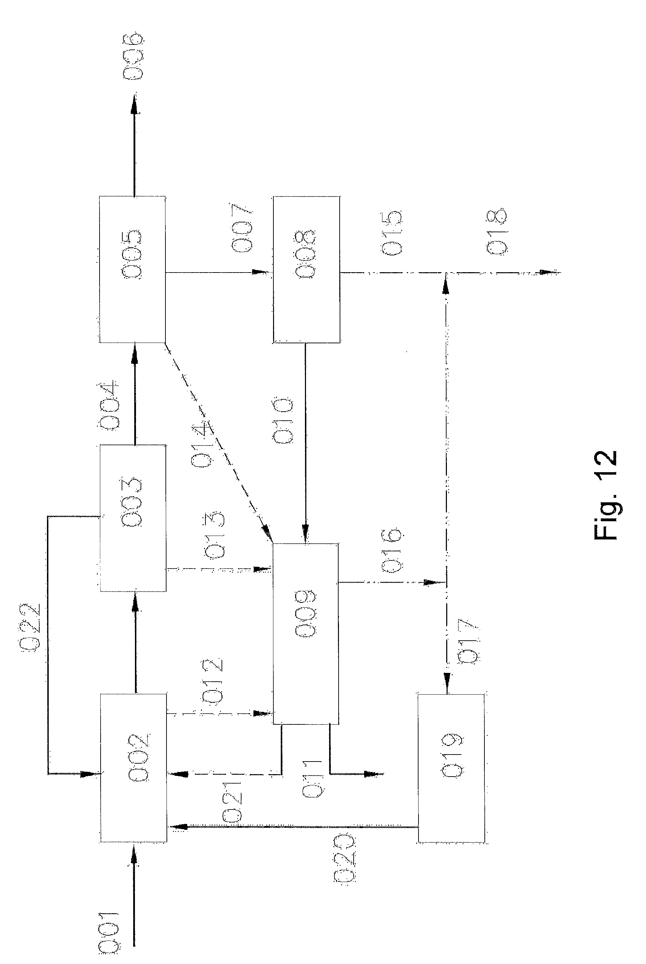

Fig. 12 is a schematic drawing of a system for producing chemical products and electricity concurrently using coal and the like as the starting material. The starting material may be coal, oil, natural gas, coke oven gas, etc.. Taking coal as an example with reference to the figure, the main process includes gasification of coal 002, purification of syngas 003, synthesis of chemical products 005, production of power with gas 008, production of power with steam 009 and air separation 019. Oxygen 020 obtained by air separation 019, steam 021 or recycle carbon dioxide 022 from purification of syngas 003 are used as the gasifying agent to produce crude syngas in the gasification of coal 002. The crude syngas is sent to the procedure of purification 003. The co-produced steam 012 during purification is sent to the steam generator 009. In the procedure of purification 003, the crude syngas is subjected to no or small CO transformation, and acidic gas impurities containing sulfur, carbon, etc., harmful to chemical synthesis and environment, are removed. The resultant purified gas 004 is sent to the procedure of chemical synthesis 005 wherein chemical products 006 such as methanol, dimethyl ether or gasoline are obtained. Steam 013 and 014 co-produced in the procedures of purification 003 and synthesis 005 is sent to the steam generator 009. Relieved syngas 007 which does not react in the procedure of synthesis 005 is sent to the gas generator 008. Steam produced in the steam generator 009 by the high temperature gas 010 from the gas generator, steam 012 from gasification 002, steam 013 from purification 003 and steam 014 from synthesis 005 are used to produce power in the steam generator 009. Fume 011 is discharged after the heat therein is recovered. Most of the power 015 from the gas generator 008 and the power 016 from the steam generator 009 is used as the output power 018, and a small part 017 is used to drive the chemical-motivational devices in air separation 019, etc..

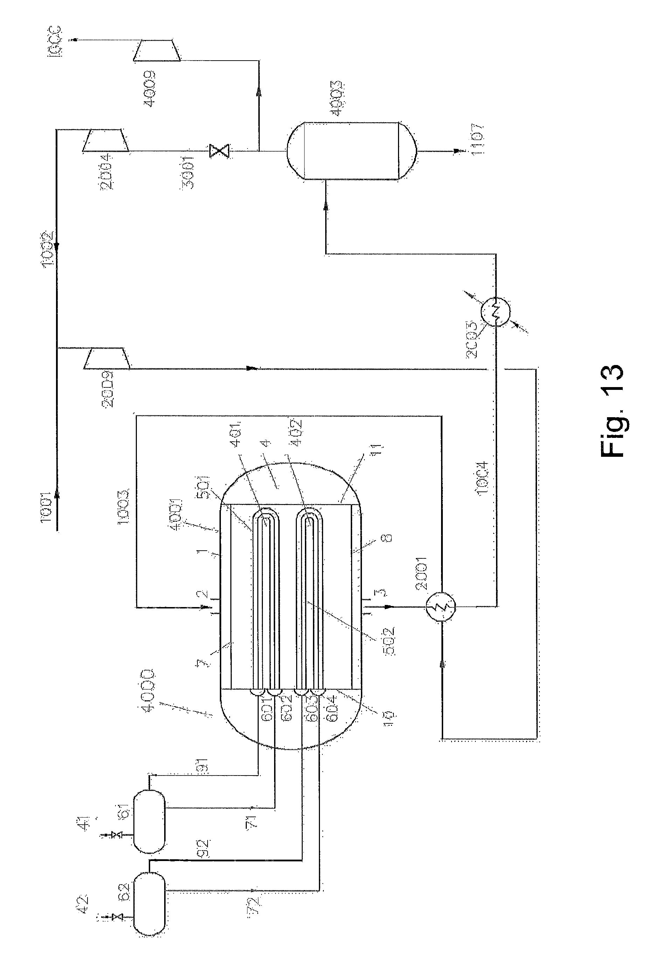

Fig. 13 is a schematic flow chart of the production of chemical products from syngas using several groups of water-cooling reactors. The equipment comprises a chemical synthesis reactor 4000 consisting of the apparatus of the invention, a gas heat exchanger 2001, a cooler 2003, a gas-liquid separator 4003, an expander 4009 and a compression cycle machine 2004. The chemical synthesis reactor 4000 is composed of a synthesis reactor 4001 and vapor tanks 61, 62 connected thereto. The synthesis reactor 4001 is composed of a shell 1, a reaction gas inlet 2 and an outlet 3 on the shell 1, partition boards 10, 11 at two opposite sides in the shell 1, a porous gas distributor 7 on the top, a porous gas collection plate 8 at the bottom, a catalyst layer 4 disposed in the space defined by the partition boards, the porous gas distributor and the porous gas collection plate, and two groups of heat-exchange tubes 501, 502 in the catalyst layer 4 for removing heat with cooling medium. The groups of heat-exchange tubes 501, 502 are composed of U-shaped tubes. The open ends of the heat-exchange tube group 501 are communicated with headers 601, 602, and the open ends of the heat-exchange tube group 502 are communicated with headers 603, 604. The headers 602, 604 are connected to the vapor tanks 61, 62 via cooling agent inlet pipes 71, 72, and the headers 601, 603 are connected to the vapor tanks 61, 62 via cooling agent inlet pipes 91, 92, so as to form circulating loops of cooling agent between the two groups of heat-exchange tubes 501, 502 and the three vapor tanks 61, 62. Steam produced by the reaction heat carried out by the heat conducting medium is discharged from valves 41, 42 for adjusting vaporization pressure. The heat conducting medium may be water, heat conducting oil or molten earth. When the latter two are used, steam may be produced by the heat-exchange tubes in the vapor tanks. The heat-exchange tube groups 501, 502 may also be composed of straight tubes, in which case both ends of the heat-exchange tubes are connected with headers. Syngas 1001 from purification 003 in Fig. 13 ( 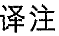 : 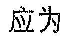 12) is pressurized by the compressor 2009, and then heated by the gas heat exchanger 2001 to produce gas 1003 which is introduced into the synthesis reactor 4001 to form methanol, dimethyl ether or gasoline depending on the catalyst used. Post-reaction gas 1004 exchanges heat via the gas heat exchanger 2002, is cooled by the cooler 2003, and then separated in the gas-liquid separator 4003 to give chemical products. The liquid chemical products 1107 are discharged from the bottom. Relieved syngas which does not react passes the expander 4009 to allow for recovery of heat before being sent to the gas generator 008 and steam generator 009 subsequently. If partial recycle is needed, recycle gas 1002 passes a pipe 3001 with a valve and the compression cycle machine 2004, and then combines with the syngas 1001 for further synthesis. The synthesis reactor 4001 in Fig. 13 may use a structure shown in any of Fig. 1-6.

The invention will be described in more detail with reference to the following examples.

### Example 1

In this example, the composite reaction apparatus of the present invention was used to synthesize methanol, wherein water was used as the cooling agent and the reaction device 9 connected to the vapor tanks 61, 62 as shown in Fig. 3 was used.

In the apparatus used in the present example as shown in Fig. 3, solid valves were closed, and hollow valves were opened. The vapor tank 61 communicated with the heat-exchange tube group 501 was vaporized at a low pressure of 0.5-5MPa, and the vapor tank 62 communicated with the heat-exchange tube groups 502, 503 was vaporized at 1-6MPa. The reaction gas proceeded the reaction in the catalyst layer 4 while it flowed downwardly. In the upper reaction portion, the reaction was fast and exhibited large reaction heat (the reaction proceeded at about 200-280°C), resulting in the temperature difference between inside and outside of the heat-exchange tube group 501 being as large as tens of degrees. In addition, the reaction heat in the catalyst layer outside the heat-exchange tube groups 502, 503 was small (the reaction there proceeded at about 220-270°C), resulting in the temperature difference between inside and outside of the heat-exchange tube groups 502, 503 being only several degrees. Thus, the temperature difference inside and outside of the heat-exchange tube group 501 was several times that of the heat-exchange tube groups 502, 503. In other words, the heat-exchange tube group 501 was located in the highly exothermic reaction zone while the heat-exchange tube groups 502, 503 were located in the weekly exothermic reaction zone. Thus, an isothermal reaction or an optimal temperature distribution can be achieved.

Into a react device having a diameter of 3.6m was loaded 80m³ of NC307 methanol catalyst. A combined gas of a syngas made from Shell coal and a recycle gas, having a pressure of 8.0 MPa, was heated to 220°C before entering the reaction device. In the upper catalytic reaction zone 411, the reaction gas reacted at about 260°C to produce methanol. The temperature of the catalytic reaction zone 411 was controlled by adjusting the vaporization pressure in the vapor tank 61 communicated with the heat-exchange tube group 501. Likewise, methanol synthesis reaction was proceeded at 250°C in the catalytic reaction zone 412, 413 by adjusting the vaporization pressure in the vapor tank 62 communicated with the heat-exchange tube groups 502, 503.

When the ratio of the recycle gas to the fresh gas was 0.5, the content of methanol in the reaction gas exiting the reaction device was 33.37%. The daily yield of methanol was 2566 tons/day, and the annual yield was 855 kilotons/year, as shown in Table 1.

**Table 1**

| Entries | | Starting Gas | Recycle Gas | Gas Exiting reaction device |
|---|---|---|---|---|
| Flow Rate Nm³/h | | 250,000 | 125,000 | 225,362 |
| Components % | H₂ | 67.16 | 77.10 | 49.85 |
| | CO | 30.03 | 11.74 | 7.66 |
| | CO₂ | 2.10 | 3.07 | 2.99 |
| | N₂+CH₃ | 0.71 | 7.69 | 5.05 |
| | H₂O | 0 | 0.07 | 1.08 |
| | CH₃OH | 0 | 0.33 | 33.37 |

When the ratio of the recycle gas to the fresh gas was reduced to 0.3, the synthesis pressure was increased to 10 MPa, and the temperature of the catalytic reaction zone 413 was changed to 200-210°C, the content of methanol in the reaction gas exiting the reaction device was increased to 50%. This content of methanol in the reaction gas was higher than that in gas-liquid equilibrium at the pressure and the temperature used in the synthesis. Thus, the excess methanol condensed from the gas phase to the liquid phase. As a result, the content of methanol in the gas phase was lowered, leading to further synthesis of methanol. In other words, the synthesis of methanol was proceeded under the conditions where methanol would be condensed, finally, the total content of methanol in the gas existing the reaction device could be increased.

### Example 2

In this example, dimethyl ether was prepared from syngas by utilizing the apparatus shown in Fig. 7. The vertical reaction device shown in Fig. 1 was used as the methanol synthesis tower which had a diameter of 4.0 m and was loaded with 120m³ of NC307 methanol catalyst. The methanol dehydration tower used to prepare dimethyl ether had a diameter of 3.0 m and was loaded with 50m³ of γ-alumina methanol dehydration catalyst. After pressurized to 8MPa, the syngas was combined with the recycle gas and heated to about 230°C before entering the methanol synthesis tower. The combined gas proceeded a reaction on the methanol catalyst layer at about 250°C to synthesize methanol. Water in the water tubes positioned horizontally in the synthesis tower absorbed the reaction heat to become steam as a byproduct. The content of methanol in the reaction gas exiting the methanol synthesis tower was 21%. This gas was combined with the recycle methanol from the rectifying tower of dimethyl ether, and then entered the methanol dehydration reactor to produce dimethyl ether. When the flow rate of the starting gas was 340,000Nm³/h, the yield of dimethyl ether was 2,500 tons/day and 830kilotons/year, as shown in Table 2.

**Table 2**

| Entries | | Starting Gas | Recycle Gas | Gas Exiting Methanol Tower | Gas Entering Dimethyl Ether Tower | Gas Exiting Dimethyl Ether Tower |
|---|---|---|---|---|---|---|
| Flow Rate 30Nm³/h | | 340,000 | 340,000 | 480,390 | 500,652 | 500,652 |
| Components % | H₂ | 67.56 | 75.38 | 58.67 | 56.30 | 56.30 |
| | CO | 29.14 | 10.66 | 8.35 | 8.01 | 8.01 |
| | CO₂ | 2.15 | 2.92 | 2.66 | 2.55 | 2.55 |
| | N₂+CH₃ | 1.14 | 10.54 | 8.23 | 7.9 | 7.9 |
| | H₂O | 0 | 0.09 | 1.0 | 0.96 | 11.08 |
| | CH₃OH | 0 | 0.4 | 21.09 | 24.28 | 4.04 |
| | (CH₃)₂O | 0 | 0 | 0 | 0 | 10.12 |

### Example 3

In this example, dimethyl ether was prepared from syngas as shown in Fig. 7. A horizontal water-cooling tower was used as the methanol synthesis tower which had a diameter of 4.0 m and was loaded with 160m³ of XN98 methanol catalyst. A vertical water-cooling tower was used as the methanol dehydration tower to prepare dimethyl ether, which had a diameter of 3.2 m and was loaded with 75m³ of γ-alumina methanol dehydration catalyst. After pressurized to 10MPa, the syngas was combined with the recycle gas and heated to about 230°C before entering the methanol synthesis tower. The combined gas reacted on the methanol catalyst layer at about 250°C to synthesize methanol. Water in the water tubes positioned horizontally in the synthesis tower absorbed the reaction heat to become steam as a byproduct. The content of methanol in the gas exiting the methanol synthesis tower was 37%. This gas was combined with the recycle methanol from the rectifying tower of dimethyl ether, heated to 300°C by heat exchange and then entered the methanol dehydration reactor to produce dimethyl ether. When the flow rate of the starting gas was 583596Nm³/h, the yield of dimethyl ether was 196tons/hour and 1560kilotons/year, as shown in Table 3.

**Table 3**

| Entries | | Starting Gas | Recycle Gas | Gas Exiting Methanol Tower | Gas Entering Dimethyl Ether Tower | Gas Exiting Dimethyl Ether Tower |
|---|---|---|---|---|---|---|
| Flow Rate Nm³/h | | 583596 | 304485 | 508000 | 525560 | 525560 |
| Components | H₂ | 65.51 | 4.05 | 25.59 | 24.74 | 24.74 |
| % | CO | 32.88 | 24.54 | 15.15 | 14.64 | 14.64 |
| | CO₂ | 1.03 | 3.80 | 3.44 | 3.33 | 3.33 |
| | N₂+CH₃ | 0.58 | 29.10 | 18.10 | 17.50 | 17.50 |
| | H₂O | 0 | 0.09 | 0.07 | 0.07 | 18.22 |
| | CH₃OH | 0 | 0.41 | 37.65 | 39.72 | 3.43 |
| | (CH₃)₂O | 0 | 0 | 0 | 0 | 18.15 |

### Example 4

The flow chart of the preparation of gasoline from syngas was shown in Fig. 8. A horizontal multistage water-cooling tower as shown in Fig.1 was used as the methanol synthesis reactor 4001 which had a diameter of 4.0 m and was loaded with 150m³ of copper-based methanol catalyst. A horizontal water-cooling tower as shown in Fig. 10 was used as the methanol dehydration reactor 4002 which had a diameter of 3.8m and was loaded with 30m³ of γ-alumina at the upper catalyst layer and 100m³ of ZSM-5 molecular seive catalyst at the middle, lower catalyst layers 402, 403. After pressurized to 6MPa, the syngas was combined with the recycle gas and heated to about 230°C before entering the methanol synthesis reactor 4001. The combined gas reacted on the methanol catalyst layer at about 250°C to synthesize methanol. Water in the water tubes positioned horizontally in the synthesis tower absorbed the reaction heat to become steam as a byproduct. The vaporization pressure in the vapor tank 61 was lower than that in the vapor tank 62 by about 1MPa. The content of methanol in the gas exiting the methanol synthesis tower was 40.2%. This gas was heated to 300°C by exchanging heat with the gas exiting the hydrocarbonation tower, and then entered the methanol dehydration reactor to produce hydrocarbons. The methanol dehydration reactor had a structure as shown in Fig. 9. The reaction gas reacted first in the upper catalytic reaction zone 411 having the water-cooling heat-exchange tube group 501 to produce dimethyl ether. Then, dimethyl ether was dehydrated at about 350°C to produce hydrocarbons in the middle, lower catalytic reaction zones 412, 413 having the heat-exchange tube groups 502, 503 using heat conducting oil. High-pressure steam of 10MPa was co-produced. When the flow rate of the starting gas was 10268 kmol/h, the yield of hydrocarbons was 110tons/day and 356kilotons/year, wherein up to 194kg hydrocarbons were obtained from 1000 Nm³ syngas, as shown in Table 4.

**Table 4**

| Entries | | Syngas | Recycle Gas | Entering Methanol Tower | Entering Hydrocarbonation Tower | Exiting Hydrocarbonation Tower | Relief gas |
|---|---|---|---|---|---|---|---|
| Flow Rate kmol/h | | 10268 | 4107 | 14375 | 7996 | 7996 | 750 |
| Components % | H₂ | 6706.4 | 2241 | 8947.3 | 2568.6 | 2568.6 | 409.2 |
| | N₂ | 18.8 | 125.1 | 143.9 | 143.9 | 143.9 | 22.8 |
| | CH₄ | 44.6 | 256.6 | 301.2 | 301.2 | 329 | 46.9 |
| | CO | 3374.9 | 1200.6 | 4575.4 | 1385.8 | 1385.8 | 219.2 |
| | CO₂ | 123.2 | 250.9 | 374.2 | 374.2 | 374.2 | 48.8 |
| | H₂O | 0 | 5.9 | 5.9 | 5.9 | 3195.4 | 1.0 |
| | CH₃OH | 0 | 26.9 | 26.9 | 3216.4 | 30.6 | 4.91 |
| | Hydrocarbons | 0 | 0 | 0 | 0 | 44580kg | |

### Example 5

The flow chart of the one-step preparation of gasoline from syngas combined with Integrated Gasification Combined Cycle (IGCC) was shown in Fig. 8. A horizontal multistage water-cooling tower as shown in Fig.1 was used as the methanol synthesis reactor 4001 which had a diameter of 3.5m and was loaded with 110m³ of copper-based methanol catalyst. A horizontal water-cooling tower as shown in Fig. 1 was used as the methanol dehydration reactor 4002 which had a diameter of 3.3m and was loaded with 90m³ of ZSM-5 molecular seive catalyst. After pressurized to 5.5MPa, the syngas was heated to 220°C before entering the methanol synthesis reactor 4001 to react on the methanol catalyst layer at about 250 °C to synthesize methanol. Water in the water tubes positioned horizontally in the synthesis tower absorbed the reaction heat to become steam as a byproduct. The content of methanol in the gas exiting the methanol synthesis reactor was 46%. This gas was heated to 300°C by exchanging heat with the gas exiting the methanol dehydration reactor 4002, and then entered the methanol dehydration reactor 4002 to produce hydrocarbons. The recycle ratio in this example was zero. The relief gas of 2772kmol/h was discharged via a purge valve 2007 and sent to Integrated Gasification Combined Cycle (IGCC) to produce power. The cycle machine 2004 in the figure was used only at the start of the operation. When the flow rate of the starting gas was 10000 kmol/h, the yield of hydrocarbons was 798.4tons/day and 265kilotons/year, wherein up to 149kg hydrocarbons were obtained from 1000 Nm³ syngas, as shown in Table 5.

**Table 5**

| Entries | | Entering Methanol Tower | Entering Hydrocarbonation Tower | Exiting Hydrocarbonation Tower | Relief gas |
|---|---|---|---|---|---|
| Flow Rate kmol/h | | 10000 | 5208.8 | 5208.8 | 2772.3 |
| Components % | H₂ | 6524.8 | 1731.7 | 1731.7 | 1718.4 |
| | N₂ | 21.7 | 21.7 | 21.7 | 21.4 |
| | CH₄ | 43.4 | 43.4 | 43.4 | 73.4 |
| | CO | 3290 | 896.3 | 896.3 | 882.0 |
| | CO₂ | 120 | 118.1 | 118.1 | 85.2 |
| | H₂O | 0 | 1.9 | 2378.2 | 4.1 |
| | CH₃OH | 0 | 2395.5 | 19.2 | 19.2 |
| | Hydrocarbons | 0 | 0 | 33268kg | |

### Example 6

The flow chart of the preparation of gasoline from syngas was shown in Fig. 8. A horizontal Fischer-Tropsch synthesis reactor as shown in Fig.1 was used as the Fischer-Tropsch synthesis reactor 4001 which had a diameter of 3.9m and was loaded with 150m³ of ultrafine Fe-Mn Fischer-Tropsch catalyst. A multistage adiabatic fixed bed reactor was used as the hydrocarbon modification reactor 4002 which had a diameter of 3.8m and was loaded with 100m³ of ZSM-5 molecular seive catalyst. After pressurized to 5MPa, the syngas was combined with the recycle gas and heated to about 210 °C before entering the Fischer-Tropsch synthesis reactor 4001 to react on the Fischer-Tropsch catalyst layer at about 240°C. Water in two groups of water tubes positioned horizontally in the reactor absorbed the reaction heat to become steam as a byproduct. The vaporization pressure in the vapor tank 61 was lower than that in the vapor tank 62 by about 1MPa. The reaction gas exiting the Fischer-Tropsch synthesis reactor was heated to 300°C by exchanging heat with the gas exiting the hydrocarbon modification reactor, and then entered the hydrocarbon modification reactor 4002. When the flow rate of the starting gas was 17544.6 kmol/h, the yield of hydrocarbons was 1739.8tons/day and 522kilotons/year, wherein up to 184.5kg hydrocarbons were obtained from 1000 Nm³ syngas, as shown in Table 6.

**Table 6**

| Entries | | Syngas | Gas Entering Tower | Gas Exiting Tower | Relief gas |
|---|---|---|---|---|---|
| Components % | Total Flow Rate | 17544.6 | 21053.6 | 10686.5 | 1983 |
| | H₂ | 11422 | 13444 | 3073.8 | 1040.7 |
| | N₂ | 137 | 394.8 | 394.8 | 137 |
| | CH₄ | 5.26 | 14.1 | 14.1 | 5.26 |
| | CO | 5769 | 6855 | 1674.5 | 586 |
| | CO₂ | 210.7 | 326 | 323 | 198.6 |
| | H₂O | 0 | 3.5 | 5206.4 | 5.5 |
| | Hydrocarbons | 0 | 0 | 72490kg/h | 0 |

### Example 7

The flow chart of the one-step preparation of gasoline from syngas combined with Integrated Gasification Combined Cycle (IGCC) was shown in Fig. 8. A horizontal Fischer-Tropsch synthesis reactor as shown in Fig.1 was used as the Fischer-Tropsch synthesis reactor 4001 which had a diameter of 5.2m and was loaded with 130m³ of iron-based or cobalt-based Fischer-Tropsch catalyst and 90m³ of ZSM-5 molecular seive catalyst. After pressurized to 5.5MPa, the syngas was heated to 220°C before entering the Fischer-Tropsch synthesis reactor 4001 to undergo Fischer-Tropsch synthesis and hydrocarbon modification on the composite Fischer-Tropsch catalyst layer at about 250°C. Water in two groups of water tubes positioned horizontally in the reactor absorbed the reaction heat to become steam as a byproduct. The vaporization pressure difference between the two groups of water tubes was about 1MPa. The conversion of CO in the gas exiting the Fischer-Tropsch synthesis reactor 4001 was 78%. The recycle ratio in this example was zero. The relief gas of3995kmol/h was discharged via a purge valve 2007 and sent to Integrated Gasification Combined Cycle (IGCC) to produce power. When the flow rate of the starting gas was 17544.46 kmol/h, the yield of hydrocarbons was 1515tons/day and 455kilotons/year, wherein up to 165kg hydrocarbons were obtained from 1000 Nm³ syngas, as shown in Table 7.

**Table 7**

| Entries | | Syngas | Gas Entering Tower | Gas Exiting Tower | Gas To IGCC |
|---|---|---|---|---|---|
| Components % | Total Flow Rate | 17544.6 | 17544.6 | 8495.8 | 3963.5 |
| | H₂ | 11422.3 | 11422.3 | 2369.6 | 2368 |
| | N₂ | 126 | 126 | 126.2 | 126 |
| | CH₄ | 8.8 | 8.8 | 8.8 | 8.8 |
| | CO | 5776.9 | 5776.9 | 1256.3 | 1256 |
| | CO₂ | 210.5 | 210.5 | 206.7 | 206.7 |
| | H₂O | 0 | 0 | 4528.7 | 6.8 |
| | Hydrocarbons | 0 | 0 | 63130kg/h | 0 |

In the above two examples, the syngas used in the Fischer-Tropsch reaction comprises inert gas <1% and H₂ + CO > 97%. Such a starting gas can be successfully used to carry out highly exothermic Fischer-Tropsch reaction, and the catalyst will not be overheated. Syngas with high content of inert gas such as N₂, CH₄ and the like can be used more safely and suitably.

### Example 8

The flow chart of the preparation of natural gas by methanation of syngas was shown in Fig. 11. A horizontal water-cooling tower as shown in Fig.1 was used as the methane synthesis tower 4001 which had a diameter of 4.1m and was loaded with 250m³ of nickel methane catalyst containing 60% Ni supported on alumina. After purified and pressurized to 5MPa, the syngas obtained in a Lurgi coal-gasification oven was heated to 290°C before entering the methane synthesis tower 4001 to react on the methanation catalyst layer at about 320°C to produce methane. The reaction heat was absorbed and carried out by the heat conducting oil in the heat-exchange tubes positioned horizontally in the synthesis tower to produce steam as a byproduct. After the gas exiting the methane synthesis tower was cooled and water was removed therefrom by separation, it comprises 94.2% CH₄. When the flow rate of the starting gas was 282584Nm³/h, the yield of man-made natural gas was 100000Nm³/h, and 350tons/hour steam was obtained as a byproduct at the same time, as shown in Table 8.

**Table 8**

| Entries | | Entering Methanation Tower | Exiting Methanation Tower | Methanation |
|---|---|---|---|---|
| Flow Rate Nm³/h | | 282584 | 162022 | 100000 |
| Components % | H₂ | 65.10 | 0.85 | 1.38 |
| | CO | 20.90 | 0.32 | 0.52 |
| | CO₂ | 1.00 | 0.67 | 1.08 |
| | CH₄ | 12.00 | 58.14 | 94.20 |
| | N₂ | 1.00 | 1.74 | 2.82 |
| | H₂O | 0 | 38.28 | 49838kg/h (liquid ) |

### Example 9

The flow chart of the synthesis of ammonia from a synthetic gas containing hydrogen and nitrogen is shown in Fig. 11. A horizontal water-cooling synthesis tower as shown in Fig. 1 was used as the reactor which had a diameter of Φ 3600 and was loaded with 50m³ of highly active ferrous oxide-based ammonia synthesis catalyst prepared according to China Patent CN 01135660. The synthetic gas (10500kmol/h) composed of 73.8% H₂, 24.6% N₂, 1.29% CH₄ and 0.31% Ar (based on molecular percentage) was put into reaction to produce ammonia at 19MPa and about 400°C with a space velocity of about 15000Nm³/h. The content of ammonia in the gas leaving the tower was greater than 20%. The yield was 2000tons/day. The co-produced high-pressure steam was 1.1tons/ton ammonia.

### Example 10

The flow chart of the transformation of carbon monoxide in syngas is shown in Fig. 11. A horizontal water-cooling synthesis tower as shown in Fig. 9 was used as the reactor which had a diameter of Φ 3500 and was loaded with 25m³ of iron-chromium transformation catalyst at the upper part and 15m³ of copper family low transformation catalyst at the lower part. The upper part of the tower had at least two groups of heat-exchange tubes which were capable of adjusting vaporization temperature and pressure independently and connected to vapor tanks 61, 62. Heat conducting oil was used in the tubes to carry reaction heat to the vapor tanks for indirect heat exchange. Alternatively, boiler water was used directly in the heat-exchange tubes to absorb heat. The transformation at the upper part was carried out at 300-380°C. Water was used in the heat-exchange tube group connected to a vapor tank 63 at the lower part to absorb heat directly. The temperature at the lower part was 200-280°C. The pressure for the transformation was 5MPa. After CO₂ was removed by the transformation and water was removed subsequently, the ratio of hydrogen to carbon of the syngas having a flow rate of 2x10⁵Nm³/h was 2, which was suitable for synthesizing methanol at a yield of 1000tons/day. The data were shown in Table 9.

**Table 9**

| Entries | | To Transformation | From Transformation |
|---|---|---|---|
| Flow Rate Nm³/h | | 200000 | 200000 |
| Components % | H₂ | 18.68 | 28.38 |
| | N₂ + Ar + CH₄ etc. | 0.6 | 0.6 |
| | CO | 23.54 | 13.84 |
| | CO₂ | 10.4 | 20.1 |
| | H₂O | 46.61 | 36.91 |

### Example 11

The flow chart of the preparation of natural gas by methanation of coke oven gas is shown in Fig. 11. A horizontal water-cooling tower 4001 as shown in Fig. 1 was used as the methane synthesis tower which had a diameter of 3.8m and was loaded with 180m³ of MoS₂ methanation catalyst supported on alumina. After purified, the coke oven gas was pressurized to 3MPa and heated to 270°C before entering the methane synthesis tower to react on the methanation catalyst layer at about 300°C. Water in the heat-exchange tubes positioned horizontally in the synthesis tower absorbed the reaction heat to produce steam as a byproduct. After cooled and with water removed, the gas exiting the methane synthesis tower contained 57.95% CH₄. When the flow rate of the starting gas was 282584 Nm³/h, the yield of man-made natural gas was 100000Nm³/h and the co-produced steam was 216tons/hour, as shown in Table 10.

**Table 10**

| Entries | | Entering Methanation Tower | Exiting Methanation Tower | Methanation |
|---|---|---|---|---|
| Flow Rate Nm³/h | | 166535 | 127900 | 100000 |
| Components % | H₂ | 59.70 | 25.72 | 32.89 |
| | CO | 7.1 | 0.86 | 1.1 |
| | CO₂ | 8.0 | 3.70 | 4.73 |
| | CH₄ | 23.2 | 45.31 | 57.95 |
| | N₂ | 2 | 2.6 | 3.33 |
| | H₂O | 0 | 21.82 | 22426Kg/h |

### Example 12

In this example, a methanol-power polygeneration system using coal-derived syngas as the starting material was illustrated as shown in Fig. 12. Coal was consumed at a rate of 31.6kg/s for the production of the starting material 001. At the beginning of gasification 002, the total availability (hereafter named exergy) of the feed including coal was 883.3MW. After purification 003, the purified syngas was subjected to synthesis 005 at a flow rate of 2.778kmol/s. The components of the gas and the flowing rates thereof are presented in Table 11. In the chemical synthesis procedure 005, the flow process shown in Fig. 13 was applied to synthesize methanol from the syngas in one pass. After pressurized to 5MPa, the syngas 1003 was heated to about 220°C by the gas 1001 exiting the tower via a heat exchanger 2001 before entering the methanol synthesis reactor 4001 to react at about 220-270°C in the presence of about 100m³ of methanol catalyst to synthesize CH₃OH from H₂ and CO, CO₂. The vaporization temperature of the water in the heat-exchange tube groups 501, 501 in the methanol synthesize reactor can be adjusted independently to keep an even temperature distribution and a small temperature difference in the methanol synthesis catalyst layer 4. The gas exiting the synthesis tower 4001, containing 25.1% methanol, exchanged heat via the heat exchanger 2002, and was cooled to about 35°C by a cooler 2003. Then, it was separated by a gas-liquid separator 4003 to give the liquid product methanol 006 and relief gas 007, wherein the yield of methanol was 14.66kg/s, and the relief gas 007 was sent to a gas generator 008 after passing an expander 4009 for energy recovery. Steam produced in the steam generator 009 by the high temperature gas 010 from the gas generator 008, steam 012 from gasification 002, steam 013 from purification 003 and steam 014 from synthesis 005 are used to produce power in the steam generator 009. Fume 011 is discharged after the heat therein is recovered. Except a small part 018 for self use, the power for output was 188MW. The data are presented in Table 12 in the column for Example 12.

**Table 11 Components of the Gas for Chemical Synthesis**

| Components | H₂ | CO | N₂ | CH₄ | CO₂ | H₂O | CH₃OH | Total |
|---|---|---|---|---|---|---|---|---|
| mol% | 40.04 | 57.21 | 2.1 | 0.06 | 0.59 | 0 | 0 | 100 |
| kmol/s | 1.122 | 1.589 | 0.058 | 0.002 | 0.016 | 0 | 0 | 2.778 |

When converted to separate production: If the same amount of crude syngas obtained by gasification 002 as in Example 12 was used to produce methanol without involving power generation, after transformation of CO and removal of CO₂ in the purification procedure 003, the hydrogen/carbon ratio of the syngas was 2. In the synthesis of methanol 005, known technology such as a Lurgi tube-shell synthesis tower may be used. The molar flow rate of the recycle gas 1002 to the starting syngas 1001 was about 5. To obtain the same yield of methanol as in this example, i.e. 14.66kg/s, an exergy of the coal used as both starting material and fuel was 665.2MW. To produce methanol solely, the energy consumed was 45.3GJ/T methanol, and the energy conversion was 48%. In contrast, the energy consumed was 30.77GJ/T in the joint production. On the other hand, when 188MW power was output in an Integrated Gasification Combined Cycle (IGCC) system for generating power solely, the exergy of starting material and fuel was calculated to be 432.2MW based on an energy conversion of 43.5%. Thus, to produce the same amount of methanol and power as in this example, the total exergy of starting material and fuel needed in Integrated Gasification Combined Cycle (IGCC) and methanol synthesis was: 665.2 + 432.2 = 1097.4MW. Therefore, when methanol and power were produced separately, the total efficiency of energy was 46.2%. In this example of joint production, the total exergy of coal as starting material and fuel was 883.3MW, and the total efficiency of energy was 57.4%. In other words, the relative energy saving ratio = 1- 883.3/1097.4 = 0.195, i.e. 19.5%. The data are presented in Table 12 in the column for Example 10.

### Example 13

In this example, a dimethyl ether-power polygeneration system using coal-derived syngas as the starting material was illustrated, and the flow process shown in Fig. 12 was used again. One difference from Example 12 was that dimethyl ether was produced in the procedure 005. The flow process shown in Fig. 13 was also applied to synthesize dimethyl ether from syngas in one pass, but about 150m³ of a two-way catalyst composed of copper-based methanol catalyst A and γ-alumina or molecular seive dehydration catalyst B was used instead of the copper-based methanol catalyst in Example 10. Synthesis of dimethyl ether was carried out at 6MPa and about 300°C. Similarly, the temperature of the catalyst in the synthesis of dimethyl ether may be adjusted independently by means of the vaporization temperature and pressure of the heat-exchange medium such as water in two groups of water-cooling heat-exchange tubes, so as to reduce the temperature difference and obtain an even temperature distribution. Without recycle, syngas having the same components and flow rates as in Table 10 at a total flow rate of 2.778kmol/s was used to produce 10.43kg/s dimethyl ether in one pass. Relief gas was sent to a gas/steam combined power plant. Except for self use, the power available for output was 157MW. The energy consumed was 46.21GJ/T dimethyl ether, and the total exergy utility was 53.59%. To obtain 10.43kg/s dimethyl ether as in this joint production, the energy consumed was 63.2GJ/T in the existing one-step synthesis of dimethyl ether for producing dimethyl ether alone. To obtain 175MW of power for output, the exergy of starting material and fuel needed in Integrated Gasification Combined Cycle (IGCC) was 402.3MW. When dimethyl ether and power were produced separately, the total exergy of starting material and fuel was 1061.3MW. The relative energy saving ratio was 16.76%. The data are presented in Table 12 in the column for Example 13.

### Example 14

In this example, a gasoline-power polygeneration system using coal-derived syngas as the starting material was illustrated, and the flow process shown in Fig. 12 as well as the reactor and the flow process shown in Fig. 13 were used. The reactor 4000 was loaded with 120m³ of iron or cobalt family Fischer-Tropsch synthesis catalyst. Syngas having the same components and flow rates as in Table 10 was used to produce 6.1kg/s gasoline at 4MPa and about 250°C. The power available for output was 172MW. The data concerning the joint production and the separate production were presented in Table 12 in the column for Example 14. The relative energy saving ratio was 15.8%.

**Table 12 Comparison of Chemical-power Polygeneration and Separate Production**

| | Example 11 | | | Example 12 | | | Example 13 | | |
|---|---|---|---|---|---|---|---|---|---|
| | Methanol -power Polygeneration | Separate Production | | Dimethyl ether -power Polygeneration | Separate Production | | Gasoline -power Polygeneration | Separate Production | |
| | | IGCC | Methanol | | IGCC | Dimethyl ether | | IGCC | Gasoline |
| Exergy of starting material and fuel MW | 883.3 | 1097.4 | | 883.3 | 1061.3 | | 883.3 | 1049.3 | |
| | | 432.2 | 665.2 | | 402.3 | 659 | | 395.4 | 653.9 |
| Product yield kg/s | 14.66 | | 14.66 | 10.43 | | 10.43 | 6.1 | | 6.1 |
| Power output MW | 188 | 188 | | 175 | 175 | | 172 | 172 | |
| Energy consumption GJ/T | 30.77 | | 45.38 | 46.21 | | 63.2 | 79.98 | | 107.2 |
| Total exergy efficiency % | 57.4 | 46.23 | | 53.59 | 44.6 | | 52.04 | 44.66 | |
| Relative energy saving ratio % | 19.5 | 0 | | 16.76 | | | 15.8 | | |

### Beneficial Effects

Compared with current arts, the invention exhibits outstanding advantages. First, different vaporization pressures and temperatures of heat-exchange medium are used in the heat-exchange tube groups in different catalyst layers. Particularly, in the front part where the reaction rate is fast and the reaction heat is large, vaporization occurs at low pressure and low temperature to enlarge the temperatures difference for heat-exchange, so that the reaction heat is removed sufficiently to prevent the catalyst from being overheated. Meanwhile, in the part where the reaction rate is slow and the reaction heat is small, vaporization occurs at relatively high pressure and temperature, lest too much heat be removed and the temperature become too low. Thus, recycle ratio is reduced to be less than half of that in current arts, and flow rate in the circulating loop of methanol synthesis is lowered by half. As a result, the size of the synthesis apparatus may be reduced greatly, leading to great reduction in investment and improved feasibility of scaling up. Secondly, since recycle ratio is reduced and the content of methanol exiting the methanol tower is up to 10%-50% which is several times higher than that obtainable in current arts (3%-6%), the electrical power consumed by the cycle machine decreases considerably. On the other hand, the reaction heat recovered and the steam co-produced per ton of methanol are increased in a large scale, whereas the cooling water in the water cooler used to cool the reaction gas is reduced significantly. Thus, energy consumption is lowered remarkably, and a notable effect in energy saving can be envisioned. Third, temperatures at different parts of the catalyst may be regulated independently according to the need of a reaction. For example, an optimal temperature curve may be obtained to achieve the fastest reaction rate in ammonia synthesis, etc.. As another example, in the case that the hot spot moves backwards during the reaction along with the degradation of the catalyst activity, vaporization pressure and temperature in the heat-exchange tubes in the upper and the lower catalyst layers may be regulated to achieve the best effect of the catalyst. Fourth, high content of methanol in reaction gas favors one-step synthesis of dimethyl ether from syngas using a fixed bed reactor. The reaction gas with a content of methanol several times higher than that in current arts may be used to produce dimethyl ether having a content of more than 10% by catalytic dehydration, facilitating separation of dimethyl ether. For annual yield of 830 kilotons dimethyl ether as in Example 5 described above, a reactor of 7.8m in diameter and 30m in height would be needed if a slurry bed reactor were used as in foreign countries. For the same reason, when the composite apparatus of the invention is used to reduce recycle ratio and produce high content of methanol, the difficult technical problems met in synthesis of hydrocarbons from syngas via methanol will be solved, and preparation of hydrocarbons by Fischer-Tropsch reaction, methanation and the like which involve highly exothermic reactions, as well as chemical-power polygeneration, low recycle synthesis and no recycle synthesis are possible.

The subject of the invention has been described with reference to the drawings and examples. According to the spirit of the invention, it is apparent for those with ordinary skills in the art that various changes may be made to the invention and used in synthesis of dimethyl ether from methanol. The apparatus of the invention may be connected to two or more vapor tanks. A water pump 81 may be communicated with the water pipe 71 connected to the vapor tank 61 in the composite reactor to reinforce the recycle of water. Alternatively, natural recycle of water may be used instead of the water pump 81. Inlet and outlet water pipes may or may not be controlled by valves, and one group of heat-exchange tubes corresponds to one vapor tank. Heat-exchange tubes may be round tubes or flat tubes, or even heat-exchange plates. Heat-exchange medium may be water, or mineral oil, heat conducting oil or molten salts.

Since the temperature is usually low at the beginning of the reaction when reaction gas enters the catalyst layer(s), an adiabatic zone may be set before the heat-exchange tube group(s) on condition that the volume of the catalyst in this adiabatic zone does not exceed one tenth of the total volume after it recovers and contracts. At the back of the catalyst, an adiabatic zone may also be set behind the heat-exchange reaction zone. In the case as described above that two genres of catalysts are disposed at the upper heat-exchange reaction zone and the lower heat-exchange reaction zone respectively, an adiabatic zone may also be set between these two zones to promote reaction temperature.

## Claims

1. A composite reaction apparatus, consisting essentially of a reaction device (9) and vapor tanks (61, 62), each of said vapor tank being capable of independently adjusting its vaporization pressure and connected fluidly with pipes (41, 42) having vaporization pressure adjusting values;
wherein said reaction device (9) comprising a capped cylindrical shell (1), a reaction gas inlet (2) and a reaction gas outlet (3) on the shell (1), a catalyst layer (4), a porous gas distributor (7), a porous gas collection plate (8) and groups of heat-exchange tube (5) in the catalyst layer (4) for removing heat via cooling agent therein;
wherein there are more than two catalytic reaction zones (411, 412.....) along the flowing direction of the reaction gas in the catalyst layer (4), at least two of the reaction zones comprise groups of heat-exchange tube (501, 502) to form heat-exchange reaction zones, respectively, each group of heat-exchange tube has an inlet pipe (302, 304) and an outlet pipe (301, 303) for cooling agent, said inlet pipe (302, 304) and outlet pipe (301, 303) are fluidly communicated with the vapor tank (61, 62), respectively, forming cooling agent circulating loops having adjustable vaporization pressure and temperature; and optionally
each of the cooling agent circulating loops comprises a recycle pump to enhance circulation;
so that the reaction gas proceeds the reaction at different heat exchanging rates sequentially in the catalytic reaction zones to obtain desirable constant temperature or optimal temperature distribution.

2. The composite reaction apparatus of Claim 1, wherein each group of the heat-exchange tube (501, 502, 503) is connected to the vapor tank (61) or the vapor tank (62) respectively, e.g. the vapor tank (62) is connected to the inlet pipes (302, 304, 306) of the groups of heat-exchange tube (501, 502, 503) in the reaction device (9) via a water pipe (72), a water pump (82) and water pipes (102, 104, 106) having valves, while the outlet pipes (301, 303, 305) of the heat-exchange tube groups (501, 502, 503) are connected to the vapor tank (62) via vapor-water pipes (101, 103, 105) having valves and a vapor-water pipe (92);
wherein adjusting the valves of each pair of pipes (101, 102), (103, 104), (105, 106) connected with the vapor tank (62) can control the flow rate or stop the flow of the cooling agent in each group of water pipes communicated with the reaction device;
wherein the vapor tank (61) is connected to the inlet pipes (302, 304, 306) of the groups of heat-exchange tube (501, 502, 503) in the reaction device (9) via a water pipe (71), a water pump (81) and water pipes (202, 204, 206) having valves, while the outlet pipes (301, 303, 305) of the groups of heat-exchange tube (501, 502, 503) connected to the vapor tank (61) via vapor-water pipes (201, 203, 205) having valves and a vapor-water pipe (91);
wherein adjusting the valves of each pair of pipes (201, 202), (203, 204), (205, 206) connected to the vapor tank (61) can control the flow rate or stop the flow of cooling agent in each group of water pipes communicated with the reaction device, and
wherein only those valves in one pair of inlet and outlet pipes that are connected to one vapor tank are opened for each group of heat-exchange tubes.

3. The composite reaction apparatus of Claim 1 or 2, wherein two or more groups of heat-exchange tubes (501, 502, 503) are connected in series, and are connected to the vapor tank (61 or 62), respectively, e.g. after the outlet pipe (303) of the heat-exchange tube group (502) and the inlet pipe (302) of the heat-exchange tube group (501) are connected via a communicating pipe (21) having a valve, the valves in the communicating pipe (21), the water pipe (104) and the vapor-water pipe (101) or the water pipe (204) and the vapor-water pipe (201) are opened, while the valves in the vapor-water pipes (103, 203) and the water pipes (102, 202) are closed, so that the two groups of heat-exchange tubes (501, 502) are connected in series and then form a circulating loop with the vapor tank (61 or 62); alternatively, after the outlet pipe (305) of the heat-exchange tube group (502) and the inlet pipe (302) of the heat-exchange tube group (501) are connected via a communicating pipe (21) with a valve, the valves in the communicating pipe (21), the water pipe (104) and the vapor-water pipe (101) or the water pipe (204) and the vapor-water pipe (201) are opened, whereas the valves in the vapor-water pipes (103, 203) and the water pipes (102, 202) are closed, so that the two groups of heat-exchange tubes (501, 502) are connected in series and then form a circulating loop with the vapor tank (61 or 62).

4. The composite reaction apparatus of Claim 1 or 2, wherein the shell (1) and the groups of heat-exchange tubes (501, 502, 503) are disposed horizontally to allow the cooling agent in the tubes to flow horizontally, and two partition plates (10, 11) are disposed at both ends of the catalyst layer (4) outside the groups of heat-exchange tubes to allow a heat-exchange reaction zone to be formed by the shell (1), the partition plates (10, 11) at both ends of the catalyst layer (4), the porous gas distributor (7) on top of the catalyst layer (4) and the porous gas collection plate (8) at the bottom thereof, wherein the reaction gas undergoes reaction while flowing vertically in the catalyst layers (401, 402, 403), and exchange heat with the heat-exchange medium flowing horizontally in the various groups of heat-exchange tubes (501, 502, 503).

5. The composite reaction apparatus of Claim 1 or 2, wherein left headers (601, 603, 605) of the heat-exchange tube groups (501, 502, 503) are connected to the inlet pipes (302, 304, 306) respectively, while headers (602, 604, 606) are connected to the outlet pipes (301, 303, 305) respectively; and right headers (701) and (702) of the heat-exchange tube group (501) are connected via a communicating pipe (801), right headers (703) and (704) of the heat-exchange tube group (502) are connected via a communicating pipe (802), and right headers (705) and (706) of the heat-exchange tube group (503) are connected via a communicating pipe (803), so as to form more than two groups of heat-exchange tubes in which the cooling agent flows counter currently in the tubes.

6. The composite reaction apparatus of Claim 1 or 2, wherein the heat-exchange tube groups (501, 502......) are composed of U-shaped tubes, the open ends of which are communicated with headers (601, 602, ..., 606), wherein the header (602) is connected to the inlet pipe (302), while the header (601) is connected to the outlet pipe (301), so as to form a heat-exchange reaction zone (411) containing the heat-exchange tube group (501); the header (602) and the header (601) are connected to the vapor tank (61) via the inlet pipe (302) and the outlet pipe (301) respectively; the header (604) and the adjacent header (605) are connected in series via a communicating pipe (800) to form a heat-exchange reaction zone (412) containing the heat-exchange tube group (502); and the header (606) and the header (603) are connected to the vapor tank (62) via the inlet pipe (304) and the outlet pipe (303) respectively.

7. The composite reaction apparatus of Claim 1 or 2, wherein the shell (1) and the groups of heat-exchange tubes (501, 502, 503) are disposed vertically to allow the heat-exchange medium in the tubes to flow vertically, and two partition boards (10, 11) are disposed at the top and the bottom ends of the catalyst layer (4) outside the groups of heat-exchange tubes to allow a heat-exchange reaction zone to be formed by the shell (1), the partition plates (10, 11) at the top and the bottom ends of the catalyst layer (4), the porous gas distributor (7) at one side of the catalyst layer (4) and the porous gas collection plate (8) at another side thereof, wherein the reaction gas undergoes reaction while flowing horizontally in the catalyst layers (411, 412, 413), and exchanges heat with the heat-exchange medium flowing vertically in the groups of heat-exchange tubes (501, 502, 503).

8. The composite reaction apparatus of Claim 1, wherein the shell (1) and the heat-exchange tubes (5) are disposed vertically to form a water-pipe type radial tower,
the outer heat-exchange tube group is composed of a plurality of lower current-dividing rings (611), upper current-collecting rings (711) and heat-exchange tubes (504) fluidly connecting said lower current-dividing rings (611) and upper current-collecting rings (711) together, said lower current-dividing rings (611) and upper current-collecting rings (711) are disposed concentrically;
the inner heat-exchange tube group is composed of a plurality of lower current-dividing rings (612), upper current-collecting rings (712) and heat-exchange tubes (505) fluidly connecting the lower current-dividing rings (612) and upper current-collecting rings (712) together, said lower current-dividing rings (612) and upper current-collecting rings (712) are disposed concentrically;
the catalyst layers (404, 405) fill in the volume outside the inner and outer heat-exchange tube groups between the peripheral porous gas collection plate (8) and the central porous gas distributor (7) in the shell (1); the lower current-dividing ring (611) below the outer heat-exchange tube group (504) is connected to the vapor tank (61) via a water pipe (71), and the upper current-collecting ring (711) above the outer heat-exchange tube group (504) is connected to the vapor tank (61) via a vapor-water pipe (91), so as to form a circulating loop of the outer heat-exchange tube group (504); the lower current-dividing ring (612) below the inner heat-exchange tube group (505) is connected to the vapor tank (62) via a water pipe (72), and the upper current-collecting ring (712) above the inner heat-exchange tube group (505) is connected to the vapor tank (62) via a vapor-water pipe (92), so as to form a circulating loop of the outer heat-exchange tube group (505);
the gas enters the reaction device (9) from the inlet (2), passes the porous gas distributor (7), flows outwardly in the catalyst layers (404, 405) outside the heat-exchange tube groups and undergoes reaction and heat-exchange therein before exiting the porous gas collection plate (8) and finally the reaction device from the outlet (3); alternatively, the functionalities of the inlet (2) and the outlet (3) may be exchanged so that the gas flows inwardly in the catalyst layers (404, 405) outside the groups of heat-exchange tubes (504, 505) and undergoes reaction and heat-exchange therein.

9. The composite reaction apparatus of Claim 1, wherein the reaction device is a vertical radial tower, wherein the shell (1) is disposed vertically, and the catalyst layer (4) in the shell (1) is divided into more than two catalytic reaction zones (411, 412), wherein at least two of the catalytic reaction zones each contains a group of heat-exchange tubes (501, 502) consisting of straight or helix tubes with heat-exchange medium therein, wherein each group of heat-exchange tubes (501, 502) are connected to the vapor tank (61) or (62) via the inlet water pipes (71, 72) and the vapor-water pipes (91, 92), wherein the vaporization pressure in the vapor tanks (61, 62) is adjustable.

10. The composite reaction apparatus of any one of Claim 1-9, wherein it can be used in such strong exothermic reactions as in preparation of methanol from syngas comprising H₂, CO and CO₂, synthesis of dimethyl ether, preparation of liquid hydrocarbons by Fischer-Tropsch reaction, methanation, preparation of hydrocarbons (ethylene, propylene, gasoline, etc.) from syngas via methanol , oxidation of o-xylene to prepare phthalic anhydride, oxidation of ethylene to synthesize ethylene oxide, hydrogenation of acetylene to prepare ethylene, hydrogenation of benzene to prepare cyclohexane, CO transformation, synthesis of ammonia, oxidation of hydrogen sulfide to prepare sulfur, etc..

11. A method for preparing dimethyl ether from syngas using the apparatus of Claim 1 or 9, comprising catalytic synthesis of methanol in the apparatus (4001), and dehydration of methanol in a methanol dehydration reactor (4002) to give dimethyl ether, wherein after separating dimethyl ether, methanol and water from the gas by heat-exchange, except for a small amount of the gas which is relieved, the remaining gas is recycled and combined with the starting syngas to be used in continuing synthesis of dimethyl ether.

12. The method of Claim 11, wherein the apparatus (4001) and the methanol dehydration reactor (4002) are connected in series, and the reaction gas (1004) from the outlet of the apparatus (4001) exchanges heat via a gas-gas heat exchanger (2002) before entering the methanol dehydration reactor (4002) to react, wherein the methanol dehydration reactor (4002) in series is a fixed bed reactor of gas-cooling cold tube type, and the gas entering the tower is used continuously to counterbalance the reaction heat of methanol dehydration via the heat-exchange tube, so as to reduce the temperature difference within the dehydration catalyst.

13. The composite reaction apparatus of any one of Claim 1-9, wherein the catalyst for methanol synthesis is disposed in the upper heat-exchange reaction zone, and the catalyst for methanol dehydration is disposed in the lower heat-exchange reaction zone, so that syngas reacts first in the upper heat-exchange reaction zone to produce methanol, and then methanol in the reaction gas enters the lower heat-exchange reaction zone to be dehydrated to produce dimethyl ether.

14. A method for preparing dimethyl ether from syngas using the composite reaction apparatus of any one of Claim 1-9, wherein a copper based methanol synthesis catalyst and a methanol dehydration catalyst such as alumina or molecular sieve in the apparatus are mixed and disposed in a plurality of heat-exchange reaction zones so that methanol synthesis and methanol dehydration are carried out at the same time.

15. A method for preparing hydrocarbons from syngas using the apparatus of Claim 1 or 9, comprising pressurization of syngas comprising hydrogen, carbon monoxide and the like, methanol synthesis or synthesis of both methanol and dimethyl ether in the apparatus without separating the reaction gas exiting the tower, methanol dehydration or dehydration of both methanol and dimethyl ether in the dehydration reactor (4002) containing a dehydration catalyst such as γ -Al₂O₃ ormolecular seive ZSM-5 at the same level of pressure to produce hydrocarbons, and separation of the final cooled reaction gas to give product hydrocarbons, water and unreacted syngas, wherein the separated syngas is sent to a gas/steam combined cycle power plant (IGCC), or used as recycle syngas, except for a small amount which is relieved, to mix with the starting syngas at a molecular ratio of less than 4, preferably less than 2, for continuing methanol synthesis followed by dehydration and product separation.

16. The composite reaction apparatus of any one of Claim 1-9, wherein the catalyst for methanol synthesis is disposed or both the catalysts for methanol synthesis and methanol dehydration to produce dimethyl ether are mixed and disposed in the upper heat-exchange reaction zone (511), and the catalysts for methanol dehydration and dimethyl ether dehydration to produce hydrocarbons are disposed in the lower heat-exchange reaction zones (512, 513), so that syngas reacts first in the upper heat-exchange reaction zone (511) at 200-290°C to produce methanol and dimethyl ether which are then dehydrated in the lower heat-exchange reaction zones (512, 513) at 260-380°C to produce hydrocarbons.

17. A method for preparing hydrocarbons by Fischer-Tropsch reaction from syngas using the apparatus of Claim 1 or 9, comprising pressurization of syngas comprising hydrogen, carbon monoxide and the like, Fischer-Tropsch reaction carried out in a Fischer-Tropsch reactor consisting of the apparatus to produce a wide fraction of relatively heavy hydrocarbons and water, cooling and separation of the reaction gas exiting the tower, or modification of the hydrocarbons in the reaction gas exiting the tower in a hydrocarbon modification reactor (4002), and then cooling and separation of the final reaction gas to give separated hydrocarbons, water and unreacted syngas, wherein the separated syngas is sent to a gas/steam combined cycle power plant (IGCC), or used as recycle syngas, except for a small amount which is relieved, to mix with the starting syngas for continuing Fischer-Tropsch reaction followed by product separation.

18. The method of Claim 17, wherein a composite Fischer-Tropsch catalyst, i.e. a mixture of a Fischer-Tropsch catalyst consisting of such transition elements as Fe, Co, Fe-Mn and a molecular sieve for hydrocarbon modification such as ZSM-5, is used in the Fischer-Tropsch reactor (4001) to carry out the Fischer-Tropsch reaction to produce hydrocarbons and modification of the hydrocarbons concurrently in the synthesis tower, so as to increase the gasoline fraction and improve the quality.

19. The method of Claim 11 or 15 or 18 for preparing dimethyl ether or hydrocarbons from syngas, wherein the reaction gas exiting the first synthesis reactor, in which methanol synthesis or dimethyl ether synthesis or Fischer-Tropsch synthesis is carried out, is heated to a higher temperature by a heat exchanger, and then undergoes methanol dehydration or dimethyl ether dehydration or hydrocarbon modification.

20. A method for preparing synthetic natural gas by methanation of coal-derived syngas or coke oven gas using the apparatus of Claim 1 or 9, comprising methanation of H₂, CO, CO₂ in the starting syngas to produce methane in the apparatus using a heat resistant catalyst containing 20%-65% Ni or a sulfur resistant catalyst containing MoS₂ supported on Al₂O₃ as the methanation catalyst, wherein high pressure steam is the byproduct of the methanation reaction.

21. A method of chemical-power polygeneration using the apparatus of Claim 1 or 9, wherein syngas (004), obtained by gasifying a carbon-containing starting material (001) with water, oxygen, carbon dioxide and the like, is first subjected to chemical synthesis (005) in the apparatus to produce such chemical products as methanol or dimethyl ether or hydrocarbons, the remaining syngas (007) left unreacted is sent to an integrated recycle generator system combining electricity generation by gas (008) and by steam (009), and the steam produced in gasification and purification of syngas and in chemical synthesis is also sent to the steam generator.

22. The method of Claim 21, wherein the syngas produced by gasification is subjected to no or small CO transformation, rendering the ratio of hydrogen to carbon (H₂/CO) in the syngas, which is to be subjected to chemical synthesis, to be 0.3-2.2, more preferably 0.4-2.0.

23. A method for synthesizing ammonia from a starting material comprising hydrogen and nitrogen using the apparatus of Claim 1 or 9, comprising synthesis of ammonia at 6-20 MPa and 310-450°C in the apparatus using an iron-family or ruthenium-family ammonia synthesis catalyst with steam of 8-15 MPa as a byproduct, wherein the synthesis is carried out at a constant or optimal temperature by using more than two groups of heat-exchange reaction zones capable of adjusting vaporization pressure and temperature independently.

24. A method for transforming carbon monoxide in syngas using the apparatus of Claim 1 or 9, comprising transformation of carbon monoxide at 1-18 MPa in the apparatus at 280-430°C when an iron-chromium family medium (high) transformation catalyst or a sulfur resistant catalyst is used, or at 180-300°C when a copper family low transformation catalyst is used, with steam of 1-15 MPa as a byproduct.
